# EUROPEAN PATENT SPECIFICATION

(11) **EP 2 393 939 B1**
(45) Date of publication and mention of the grant of the patent: **17.09.2014**
(21) Application number: 10703599.0
(22) Date of filing: 05.02.2010
(51) Int. Cl.: C12Q 1/68

(54) **A SNP MARKER OF BREAST AND OVARIAN CANCER RISK**
SNP-MARKER FÜR MAMMA- UND OVARIALKARZINOMRISIKO
MARQUEUR SNP DE RISQUE DU CANCER DU SEIN ET DES OVAIRES

(30) Priority: 07.12.2009 US 267284 P; 06.02.2009 US 150645 P
(43) Date of publication of application: 14.12.2011
(73) Proprietor: Yale University, New Haven, CT 06511 (US)
(72) Inventor: WEIDHAAS, Joanne B., Westport, CT 06880 (US)
(74) Representative: Mintz Levin Cohn Ferris Glovsky and Popeo LLP
(86) International application number: PCT/US2010/023412
(87) International publication number: WO 2010/101696

(56) References cited:
- EP-A1- 0 699 754
- WO-A1-2008/151004
- WO-A1-2009/155100
- RHEI E ET AL: "Molecular genetic characterization of BRCA1- and BRCA2-linked hereditary ovarian cancers." CANCER RESEARCH 1 AUG 1998 LNKD- PUBMED:9699640, vol. 58, no. 15, 1 August 1998 (1998-08-01), pages 3193-3196, XP002586576 ISSN: 0008-5472
- LI A J ET AL: "Genetic factors in ovarian carcinoma." CURRENT ONCOLOGY REPORTS JAN 2001 LNKD- PUBMED:11123866, vol. 3, no. 1, January 2001 (2001-01), pages 27-32, XP002586577 ISSN: 1523-3790
- BELL DEBRA A: "Origins and molecular pathology of ovarian cancer" MODERN PATHOLOGY, vol. 18, no. Suppl. 2, February 2005 (2005-02), pages S19-S32, XP002586578 ISSN: 0893-3952
- VON LINTIG FRIEDERIKE C ET AL: "Ras activation in human breast cancer" BREAST CANCER RESEARCH AND TREATMENT, vol. 62, no. 1, July 2000 (2000-07), pages 51-62, XP002586579 ISSN: 0167-6806
- GERMAN CONSORTIUM FOR HEREDITARY BREAST AND OVARIAN CANCER: "Comprehensive analysis of 989 patients with breast or ovarian cancer provides BRCA1 and BRCA2 Mutation Profiles and Frequencies for the German Population" INTERNATIONAL JOURNAL OF CANCER, JOHN WILEY & SONS, INC, UNITED STATES, SWITZERLAND, GERMANY LNKD- DOI:10.1002/IJC.1626, vol. 97, 1 January 2002 (2002-01-01), pages 472-480, XP002195298 ISSN: 0020-7136
- WEIDHAAS J: "Polymorphisms in miRNA binding sites as risk factors for cancer development" CANCER PREVENTION RESEARCH 2010;3(1 SUPPL):CN07-03, 6 December 2009 (2009-12-06), - 9 December 2009 (2009-12-09) XP002586580 AACR INTERNATIONAL CONFERENCE ON FRONTIERS IN CANCER PREVENTION RESEARCH, HOUSTON, TX, USA

## Description

### FIELD OF THE INVENTION

This invention relates generally to the fields of cancer and molecular biology. The invention provides methods for predicting increased risk of developing hereditary breast ovarian syndrome.

### BACKGROUND OF THE INVENTION

The genetics of Breast/Ovarian Cancer Syndrome is autosomal dominant with reduced penetrance. In simple terms, this means that the syndrome runs through families: (1) both sexes can be carriers (mostly women get the disease but men can both pass it on and occasionally get breast cancer); (2) most generations will likely have breast cancer; (3) occasionally women carriers either die young before they have the time to manifest disease (and yet have offspring who get it) or they never develop breast or ovarian cancer and die of old age (the latter people are said to have "reduced penetrance" because they never develop cancer). Pedigree analysis and genetic counseling is absolutely essential to the proper workup of a family prior to any lab work.

In 1994, the first gene associated with breast cancer, BRCA1 (BReast CAncer1) was identified on chromosome 17. EP 0 699 754 A1 (1996-03-06) teaches that some mutant alleles of the BRCA1 gene cause susceptibility to breast and ovarian cancer. A year later, a second gene associated with breast cancer, BRCA2, was discovered on chromosome 13. When individuals carry a mutated form of either BRCA1 or BRCA2, they have an increased risk of developing breast or ovarian cancer at some point in their lives. Not all hereditary breast and ovarian cancers are caused by BRCA1 and BRCA2.

Accordingly, there is a need for the identification of genetic markers that indicate a predisposition for developing cancer, e.g., ovarian cancer and/or breast cancer, that can be used to identify subjects that have an increased susceptibility for developing cancer, i.e., they are predisposed to develop cancer. Even though there has been progress in the field of cancer detection, there still remains a need in the art for the identification of new genetic markers for a variety of cancers that can be easily used in clinical applications.

### SUMMARY OF THE INVENTION

The disclosure provides a genetic test for predicting the risk of an individual developing breast cancer, ovarian cancer, and hereditary breast/ovarian syndrome. The current test for these conditions is BRCA, a time-consuming test which is predictive of hereditary breast and ovarian cancer risk in about 5 percent of individuals, most of whom are of Ashkenazi Jewish descent. Methods of the disclosure provide a simpler test, which determines the presence or absence of the LCS6-SNP (also known as the KRAS-Variant). Studies show that the KRAS-Variant is found in up to 27% of sporadic ovarian cancer. Critically, the KRAS-Variant is present in 61 % of BRCA-negative ovarian cancer patients. Thus, the disclosure represents a breakthrough method of diagnosing and prognosing patients from whom, until now, a genetic test predictive of cancer risk was unavailable. Moreover, the presence of the KRAS-Variant modifies the effect on BRCA1. The presence of both the KRAS-Variant and a BRCA1 mutation results in an increased the risk of that patient developing both breast and ovarian cancers. Also critically, the presence of the KRAS-Variant in ovarian cancer is a biomarker and predictor of poor prognosis because the presence of the KRAS-Variant is associated with more advanced stages of the disease, non-responsive forms of the disease, and decreased patient survival.

Specifically, the invention provides a method of predicting an increased risk of hereditary breast/ovarian cancer syndrome (HBOC syndrome or HBOS) in a subject, comprising detecting a single nucleotide polymorphism (SNP) at position 4 of the let-7 complementary site 6 of KRAS in a patient sample, the subject is BRCA1 or BRCA2 positive, and wherein the presence of the SNP indicates an increased risk of HBOC syndrome in the subject. Furthermore, in certain embodiments of this method, the subject is of non-Jewish or non-Ashkenazi Jewish descent.

The disclosure further provides a method of predicting an increased risk of developing ovarian cancer or breast cancer in a subject, including detecting a BRCA1 mutation and a single nucleotide polymorphism (SNP) at position 4 of the *let-7* complementary site 6 of KRAS in a patient sample wherein the presence of the BRCA1 mutation and the SNP indicates an increased risk of developing breast or ovarian cancer. In one embodiment of this method, the subject has hereditary breast/ovarian syndrome (HBOS, or HBOC syndrome). In another embodiment, the subject is BRCA2 negative. Alternatively, or in addition, the subject is of non-Jewish or non-Ashkenazi Jewish descent. In certain embodiments, BRCA1 mutations of this method are non-founder mutations.

The disclosure also provides a method of predicting an increased risk of developing both breast and ovarian cancer in a subject having HBOS (or HBOC syndrome) including detecting a BRCA1 mutation and a single nucleotide polymorphism (SNP) at position 4 of the *let-7* complementary site 6 of KRAS in a patient sample wherein the presence of the BRCA1 mutation and the SNP indicates an increased risk of developing both breast and ovarian cancer. In certain aspects of this method the subject is of non-Jewish or non- Ashkenazi Jewish descent. In other aspects of this method, BRCA1 mutations of this method are non-founder mutations.

### BRIEF DESCRIPTION OF THE DRAWINGS

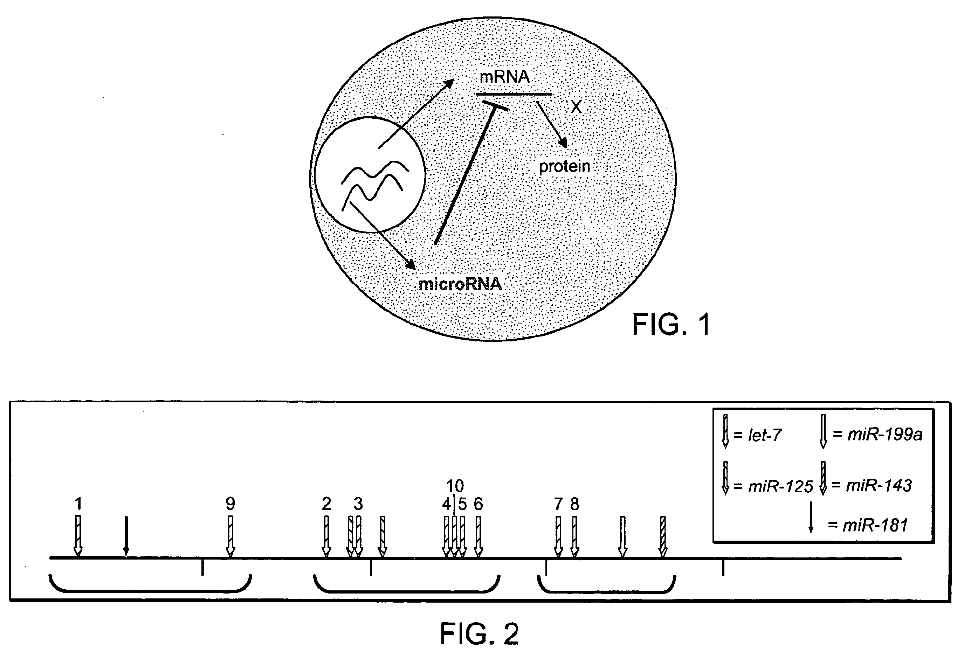

Figure 1 is an illustration depicting a new paradigm of miRNA-mediate gene regulation in which a miRNA binds a target mRNA transcript, thereby preventing translation of the mRNA into a protein.
Figure 2 is a schematic representation of let-7 family miRNA binding sites within the KRAS 3' untranslated region (3'UTR). Numbered arrows represent let-7 binding sites.
Figure 3 is a graph of the relative frequency of the KRAS-Variant occurring among various ethnic groups, wherein a thymine is substituted at a single nucleotide polymorphism (SNP) site within the sixth let-7 complementary site (LCS6) of KRAS. Overall, G allele frequency is less than 3% (sampled 4686 chromosomes). Population 1 (hatched dark grey, Blaka through Ethiopians), Population 2 (dark grey, Yemenites through Khanty), Population 3 (medium grey, Kerallte through Atayal), and Population 4 (light grey, Cheyenne though Karitiana).
Figure 4A is a graph of the relative frequencies of the KRAS- Variant, BRCA1, and BRCA2 mutations occurring among various patient groups organized by ethnic background (Jewish group), cancer diagnosis (breast and ovarian, lung/throat cancer, colon/stomach, and pancreas groups), and age.
Figure 4B is a graph of the relative frequencies of the KRAS-Variant (medium grey, right), BRCA1 (light grey, left), and BRCA2 (dark grey, middle) mutations occurring among various patient groups organized by ethnic background (Jewish group), cancer diagnosis (lung/Head and Neck (H&N) cancer), and age.
Figure 5 is a graph depicting the prevalence of the KRAS- Variant in patients with newly diagnosed epithelial ovarian cancer from Yale University, a Northern Italian Cohort, and a second Italian cohort, compared to control subjects from Yale University. The KRAS-Variant occurred in 27% of Yale patients, 26% of Northern Italian Patients (Italian 1), 25% of the Second Italian Cohort (Italian 2) and 12% of Yale Controls. Critically, the KRAS-Variant is occurs in up to 27% of ovarian cancer patients.

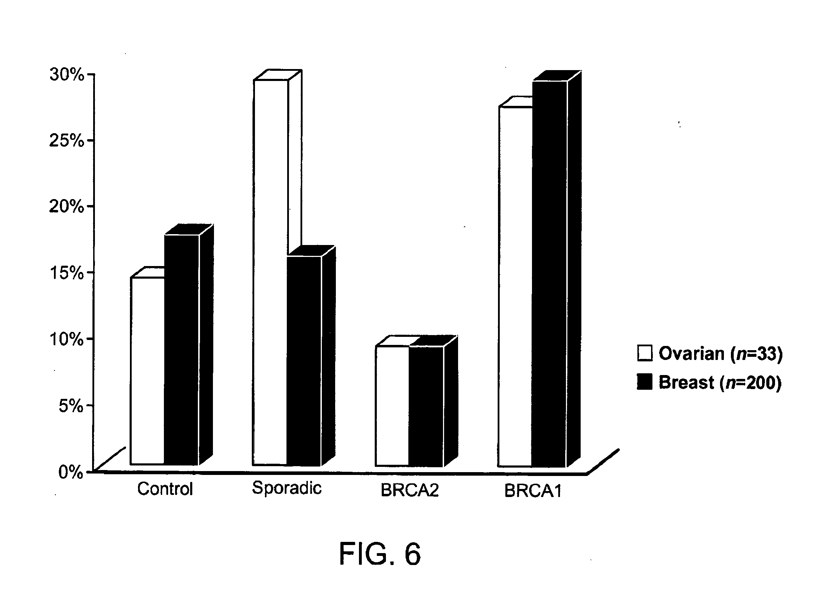

Figure 6 is a graph depicting the prevalence of the KRAS-Variant in those patients who also carry either the BRCA1 or the BRCA2 mutation. The KRAS-Variant, is more prevalent in those patients who carry the BRCA1 mutation than in patients who carry the BRCA2 mutation.
Figure 7 is a graphical depiction of a family tree, in which those members who were tested for the KRAS-Variant, and who also carry the KRAS-Variant are marked with a star.

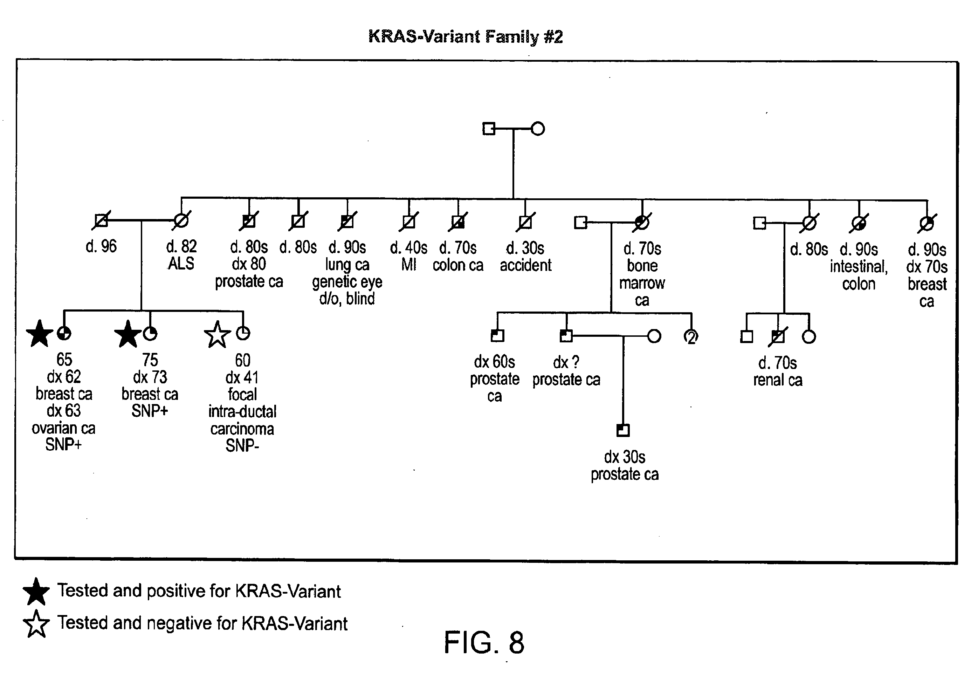

Figure 8 is a graphical depiction of a family tree, in which those members who were tested for the KRAS-Variant, and who also carry the KRAS-Variant are marked with a red (or dark gray) star. Those members who were tested for the KRAS-Variant, and who do not carry the KRAS-Variant are marked with a light gray star.

### DETAILED DESCRIPTION

The invention and disclosures herein are based upon the unexpected discovery that the presence of a SNP in the 3' untranslated region (UTR) of KRAS, referred to herein as the "LCS6 SNP," or the "KRAS-Variant" is predictive of Hereditary Breast/Ovarian Syndrome (HBOS, also known as Hereditary Breast/Ovarian Cancer (HBOC) syndrome ).

Hereditary breast ovarian cancer (HBOC) syndrome is a syndrome that causes female carriers to be at increased risk for both breast and ovarian cancer. Risk factors for this syndrome include: an early age of onset of breast cancer (often before age 50); family history of breast and/or ovarian cancer; increased chance of bilateral cancers (cancer that develop in both breasts, or both ovaries, independently) or an individual with both breast and ovarian cancer; an autosomal dominant pattern of inheritance (vertical transmission through either the mother or father's side of the family). Other factors that increase the chance that a family has the hereditary breast ovarian cancer syndrome include: family history of male breast cancer or Ashkenazi Jewish ancestry.

In 1990, DNA linkage studies on large families with the above characteristics identified the first gene associated with breast cancer. Scientists named this gene "breast cancer 1" or BRCA1. BRCA1 is located on chromosome 17. Mutations in the gene are transmitted in an autosomal dominant pattern in a family. Since it was clear that not all breast cancer families were linked to BRCA1, studies continued and in 1994, scientists discovered another gene (similar to BRCA1), and named it BRCA2. BRCA2 is located on chromosome 13. Mutations in this gene are also transmitted in an autosomal dominant pattern in a family. Both BRCA1 and BRCA2 are tumor suppressor genes that usually have the job of controlling cell growth and cell death. Everyone has two BRCA1 (one on each chromosome #17) and two BRCA2 genes (one on each chromosome #13). When a person has one altered or mutated copy of either the BRCA1 or BRCA2 gene, their risk for various types of cancer increases (U.S. Patent No. 6,051,379; 6,083,698; 6,492,109; and 7,250,497). However, at least one-third of breast cancers which seem to run in families are not linked to BRCA1 or BRCA2, suggesting the existence of an additional hereditary breast cancer gene or genes.

There are three human RAS genes comprising HRAS, KRAS, and NRAS. Each gene comprises multiple miRNA complementary sites in the 3'UTR of their mRNA transcripts. Specifically, each human RAS gene comprises multiple let-7 complementary sites (LCSs). The let-7 family-of-microRNAs (miRNAs) are global genetic regulators important in controlling lung cancer oncogene expression by binding to the 3'UTRs (untranslated regions) of their target messenger RNAs (mRNAs).

Specifically, the term "*let-7* complementary site" is meant to describe any region of a gene or gene transcript that binds a member of the *let-7* family of miRNAs. Moreover, this term encompasses those sequences within a gene or gene transcript that are complementary to the sequence of a *let-7* family miRNA. The term "complementary" describes a threshold of binding between two sequences wherein a majority of nucleotides in each sequence are capable of binding to a majority of nucleotides within the other sequence in trans.

The Human KRAS 3' UTR comprises 8 LCSs named LCS1-LCS8, respectively. For the following sequences, thymine (T) may be substituted for uracil (U). LCS1 comprises the sequence GACAGUGGAAGUUUUUUUUUCCUCG (SEQ ID NO: 1). LCS2 comprises the sequence AUUAGUGUCAUCUUGCCUC (SEQ ID NO: 2). LCS3 comprises the sequence AAUGCCCUACAUCUUAUUUUCCUCA (SEQ ID NO: 3). LCS4 comprises the sequence GGUUCAAGCGAUUCUCGUGCCUCG (SEQ ID NO: 4). LCS5 comprises the sequence GGCUGGUCCGAACUCCUGACCUCA (SEQ ID NO: 5). LCS6 comprises the sequence GAUUCACCCACCUUGGCCUCA (SEQ ID NO: 6). LCS7 comprises the sequence GGGUGUUAAGACUUGACACAGUACCUCG (SEQ ID NO: 7). LCS8 comprises the sequence AGUGCUUAUGAGGGGAUAUUUAGGCCUC (SEQ ID NO: 8).

Human KRAS has two wild type forms, encoded by transcripts a and b, which provided below as SEQ ID NOs: 9 and 10, respectively. The sequences of each human KRAS transcript, containing the LCS6 SNP (KRAS-Variant), are provided below as SEQ ID NOs: 11 and 12.

Human KRAS, transcript variant a, is encoded by the following mRNA sequence (NCBI Accession No. NM_033360 and SEQ ID NO: 9) (untranslated regions are bolded, LCS6 is underlined):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat

481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gagagtggag gatgcttttt atacattggt
661 gagggagatc cgacaataca gattgaaaaa aatcagcaaa gaagaaaaga ctcctggctg
721 tgtgaaaatt aaaaaatgca ttataatgta atctgggtgt tgatgatgcc ttctatacat
781 tagttcgaga aattcgaaaa cataaagaaa agatgagcaa agatggtaaa aagaagaaaa
841 agaagtcaaa gacaaagtgt gtaattatgt aaatacaatt tgtacttttt tcttaaggca
901 tactagtaca agtggtaatt tttgtacatt acactaaatt attagcattt gttttagcat
961 tacctaattt ttttcctgct ccatgcagac tgttagcttt taccttaaat gcttatttta
1021 aaatgacagt ggaagttttt ttttcctcta agtgccagta ttcccagagt tttggttttt
1081 gaactagcaa tgcctgtgaa aaagaaactg aatacctaag atttctgtct tggggttttt
1141 ggtgcatgca gttgattact tcttattttt cttaccaatt gtgaatgttg gtgtgaaaca
1201 aattaatgaa gcttttgaat catccctatt ctgtgtttta tctagtcaca taaatggatt
1261 aattactaat ttcagttgag accttctaat tggtttttac tgaaacattg agggaacaca
1321 aatttatggg cttcctgatg atgattcttc taggcatcat gtcctatagt ttgtcatccc
1381 tgatgaatgt aaagttacac tgttcacaaa ggttttgtct cctttccact gctattagtc
1441 atggtcactc tccccaaaat attatatttt ttctataaaa agaaaaaaat ggaaaaaaat
1501 tacaaggcaa tggaaactat tataaggcca tttccttttc acattagata aattactata
1561 aagactccta atagcttttc ctgttaaggc agacccagta tgaaatgggg attattatag
1621 caaccatttt ggggctatat ttacatgcta ctaaattttt ataataattg aaaagatttt
1681 aacaagtata aaaaattctc ataggaatta aatgtagtct ccctgtgtca gactgctctt
1741 tcatagtata actttaaatc ttttcttcaa cttgagtctt tgaagatagt tttaattctg
1801 cttgtgacat taaaagatta tttgggccag ttatagctta ttaggtgttg aagagaccaa
1861 ggttgcaagg ccaggccctg tgtgaacctt tgagctttca tagagagttt cacagcatgg
1921 actgtgtccc cacggtcatc cagtgttgtc atgcattggt tagtcaaaat ggggagggac
1981 tagggcagtt tggatagctc aacaagatac aatctcactc tgtggtggtc ctgctgacaa
2041 atcaagagca ttgcttttgt ttcttaagaa aacaaactct tttttaaaaa ttacttttaa
2101 atattaactc aaaagttgag attttggggt ggtggtgtgc caagacatta attttttttt
2161 taaacaatga agtgaaaaag ttttacaatc tctaggtttg gctagttctc ttaacactgg
2221 ttaaattaac attgcataaa cacttttcaa gtctgatcca tatttaataa tgctttaaaa
2281 taaaaataaa aacaatcctt ttgataaatt taaaatgtta cttattttaa aataaatgaa
2341 gtgagatggc atggtgaggt gaaagtatca ctggactagg aagaaggtga cttaggttct
2401 agataggtgt cttttaggac tctgattttg aggacatcac ttactatcca tttcttcatg
2461 ttaaaagaag tcatctcaaa ctcttagttt ttttttttta caactatgta atttatattc
2521 catttacata aggatacact tatttgtcaa gctcagcaca atctgtaaat ttttaaccta
2581 tgttacacca tcttcagtgc cagtcttggg caaaattgtg caagaggtga agtttatatt
2641 tgaatatcca ttctcgtttt aggactcttc ttccatatta gtgtcatctt gcctccctac
2701 cttccacatg ccccatgact tgatgcagtt ttaatacttg taattcccct aaccataaga
2761 tttactgctg ctgtggatat ctccatgaag ttttcccact gagtcacatc agaaatgccc
2821 tacatcttat ttcctcaggg ctcaagagaa tctgacagat accataaagg gatttgacct
2881 aatcactaat tttcaggtgg tggctgatgc tttgaacatc tctttgctgc ccaatccatt
2941 agcgacagta ggatttttca aacctggtat gaatagacag aaccctatcc agtggaagga
3001 gaatttaata aagatagtgc tgaaagaatt ccttaggtaa tctataacta ggactactcc
3061 tggtaacagt aatacattcc attgttttag taaccagaaa tcttcatgca atgaaaaata
3121 ctttaattca tgaagcttac tttttttttt tggtgtcaga gtctcgctct tgtcacccag
3181 gctggaatgc agtggcgcca tctcagctca ctgcaacctc catctcccag gttcaagcga
3241 ttctcgtgcc tcggcctcct gagtagctgg gattacaggc gtgtgccact acactcaact
3301 aatttttgta tttttaggag agacggggtt tcaccctgtt ggccaggctg gtctcgaact
3361 cctgacctca agtgattcac ccaccttggc ctcataaacc tgttttgcag aactcattta
3421 ttcagcaaat atttattgag tgcctaccag atgccagtca ccgcacaagg cactgggtat
3481 atggtatccc caaacaagag acataatccc ggtccttagg tagtgctagt gtggtctgta
3541 atatcttact aaggcctttg gtatacgacc cagagataac acgatgcgta ttttagtttt
3601 gcaaagaagg ggtttggtct ctgtgccagc tctataattg ttttgctacg attccactga
3661 aactcttcga tcaagctact ttatgtaaat cacttcattg ttttaaagga ataaacttga

3721 ttatattgtt tttttatttg gcataactgt gattctttta ggacaattac tgtacacatt
3781 aaggtgtatg tcagatattc atattgaccc aaatgtgtaa tattccagtt ttctctgcat
3841 aagtaattaa aatatactta aaaattaata gttttatctg ggtacaaata aacaggtgcc
3901 tgaactagtt cacagacaag gaaacttcta tgtaaaaatc actatgattt ctgaattgct
3961 atgtgaaact acagatcttt ggaacactgt ttaggtaggg tgttaagact tacacagtac
4021 ctcgtttcta cacagagaaa gaaatggcca tacttcagga actgcagtgc ttatgagggg
4081 atatttaggc ctcttgaatt tttgatgtag atgggcattt ttttaaggta gtggttaatt
4141 acctttatgt gaactttgaa tggtttaaca aaagatttgt ttttgtagag attttaaagg
4201 gggagaattc tagaaataaa tgttacctaa ttattacagc cttaaagaca aaaatccttg
4261 ttgaagtttt tttaaaaaaa gctaaattac atagacttag gcattaacat gtttgtggaa
4321 gaatatagca gacgtatatt gtatcatttg agtgaatgtt cccaagtagg cattctaggc
4381 tctatttaac tgagtcacac tgcataggaa tttagaacct aacttttata ggttatcaaa
4441 actgttgtca ccattgcaca attttgtcct aatatataca tagaaacttt gtggggcatg
4501 ttaagttaca gtttgcacaa gttcatctca tttgtattcc attgattttt tttttcttct
4561 aaacattttt tcttcaaaca gtatataact ttttttaggg gatttttttt tagacagcaa
4621 aaactatctg aagatttcca tttgtcaaaa agtaatgatt tcttgataat tgtgtagtaa
4681 tgttttttag aacccagcag ttaccttaaa gctgaattta tatttagtaa cttctgtgtt
4741 aatactggat agcatgaatt ctgcattgag aaactgaata gctgtcataa aatgaaactt
4801 tctttctaaa gaaagatact cacatgagtt cttgaagaat agtcataact agattaagat
4861 ctgtgtttta gtttaatagt ttgaagtgcc tgtttgggat aatgataggt aatttagatg
4921 aatttagggg aaaaaaaagt tatctgcaga tatgttgagg gcccatctct cccccacac
4981 ccccacagag ctaactgggt tacagtgttt tatccgaaag tttccaattc cactgtcttg
5041 tgttttcatg ttgaaaatac ttttgcattt ttcctttgag tgccaatttc ttactagtac
5101 tatttcttaa tgtaacatgt ttacctggaa tgtattttaa ctatttttgt atagtgtaaa
5161 ctgaaacatg cacattttgt acattgtgct ttcttttgtg ggacatatgc agtgtgatcc
5221 agttgttttc catcatttgg ttgcgctgac ctaggaatgt tggtcatatc aaacattaaa
5281 aatgaccact cttttaattg aaattaactt ttaaatgttt ataggagtat gtgctgtgaa
5341 gtgatctaaa atttgtaata tttttgtcat gaactgtact actcctaatt attgtaatgt
5401 aataaaaata gttacagtga caaaaaaaaa aaaaaa

Human KRAS, transcript variant b, is encoded by the following mRNA sequence (NCBI Accession No. NM_004985 and SEQ ID NO: 10)(untranslated regions are bolded, LCS6 is underlined):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gggtgttgat gatgccttct atacattagt
661 tcgagaaatt cgaaaacata aagaaaagat gagcaaagat ggtaaaaaga agaaaaagaa
721 gtcaaagaca aagtgtgtaa ttatgtaaat acaatttgta ctttttcttt aaggcatact
781 agtacaagtg gtaatttttg tacattacac taaattatta gcatttgttt tagcattacc
841 taattttttt cctgctccat gcagactgtt agcttttacc ttaaatgctt attttaaaat
901 gacagtggaa gttttttttt cctctaagtg ccagtattcc cagagttttg gtttttgaac
961 tagcaatgcc tgtgaaaaag aaactgaata cctaagattt ctgtcttggg gtttttggtg
1021 catgcagttg attacttctt atttttctta ccaattgtga atgttggtgt gaaacaaatt

1081 aatgaagctt ttgaatcatc cctattctgt gttttatcta gtcacataaa tggattaatt
1141 actaatttca gttgagacct tctaattggt ttttactgaa acattgaggg aacacaaatt
1201 tatgggcttc ctgatgatga ttcttctagg catcatgtcc tatagtttgt catccctgat
1261 gaatgtaaag ttacactgtt cacaaaggtt ttgtctcctt tccactgcta ttagtcatgg
1321 tcactctccc caaaatatta tattttttct ataaaaagaa aaaaatggaa aaaaattaca
1381 aggcaatgga aactattata aggccatttc cttttcacat tagataaatt actataaaga
1441 ctcctaatag cttttcctgt taaggcagac ccagtatgaa atggggatta ttatagcaac
1501 cattttgggg ctatatttac atgctactaa atttttataa taattgaaaa gatttaaca
1561 agtataaaaa attctcatag gaattaaatg tagtctccct gtgtcagact gctctttcat
1621 agtataactt taaatctttt cttcaacttg agtctttgaa gatagtttta attctgcttg
1681 tgacattaaa agattatttg ggccagttat agcttattag gtgttgaaga gaccaaggtt
1741 gcaaggccag gccctgtgtg aacctttgag ctttcataga gagtttcaca gcatggactg
1801 tgtccccacg gtcatccagt gttgtcatgc attggttagt caaaatgggg agggactagg
1861 gcagtttgga tagctcaaca agatacaatc tcactctgtg gtggtcctgc tgacaaatca
1921 agagcattgc ttttgtttct taagaaaaca aactcttttt taaaaattac ttttaaatat
1981 taactcaaaa gttgagattt tggggtggtg gtgtgccaag acattaattt tttttttaaa
2041 caatgaagtg aaaaagtttt acaatctcta ggtttggcta gttctcttaa cactggttaa
2101 attaacattg cataaacact tttcaagtct gatccatatt taataatgct ttaaaataaa
2161 aataaaaaca atccttttga taaatttaaa atgttactta ttttaaaata aatgaagtga
2221 gatggcatgg tgaggtgaaa gtatcactgg actaggaaga aggtgactta ggttctagat
2281 aggtgtcttt taggactctg attttgagga catcacttac tatccatttc ttcatgttaa
2341 aagaagtcat ctcaaactct tagttttttt tttttacaac tatgtaattt atattccatt
2401 tacataagga tacacttatt tgtcaagctc agcacaatct gtaaattttt aacctatgtt
2461 acaccatctt cagtgccagt cttgggcaaa attgtgcaag aggtgaagtt tatatttgaa
2521 tatccattct cgttttagga ctcttcttcc atattagtgt catcttgcct ccctaccttc
2581 cacatgcccc atgacttgat gcagttttaa tacttgtaat tcccctaacc ataagattta
2641 ctgctgctgt ggatatctcc atgaagtttt cccactgagt cacatcagaa atgccctaca
2701 tcttatttcc tcagggctca agagaatctg acagatacca taaagggatt tgacctaatc
2761 actaattttc aggtggtggc tgatgctttg aacatctctt tgctgcccaa tccattagcg
2821 acagtaggat ttttcaaacc tggtatgaat agacagaacc ctatccagtg gaaggagaat
2881 ttaataaaga tagtgctgaa agaattcctt aggtaatcta taactaggac tactcctggt
2941 aacagtaata cattccattg ttttagtaac cagaaatctt catgcaatga aaaatacttt
3001 aattcatgaa gcttactttt tttttttggt gtcagagtct cgctcttgtc acccaggctg
3061 gaatgcagtg gcgccatctc agctcactgc aacctccatc tcccaggttc aagcgattct
3121 cgtgcctcgg cctcctgagt agctgggatt acaggcgtgt gccactacac tcaactaatt
3181 tttgtatttt taggagagac ggggtttcac cctgttggcc aggctggtct cgaactcctg
3241 acctcaagtg attcacccac cttggcctca taaacctgtt ttgcagaact catttattca
3301 gcaaatattt attgagtgcc taccagatgc cagtcaccgc acaaggcact gggtatatgg
3361 tatccccaaa caagagacat aatcccggtc cttaggtagt gctagtgtgg tctgtaatat
3421 cttactaagg cctttggtat acgacccaga gataacacga tgcgtatttt agttttgcaa
3481 agaaggggtt tggtctctgt gccagctcta taattgtttt gctacgattc cactgaaact
3541 cttcgatcaa gctactttat gtaaatcact tcattgtttt aaaggaataa acttgattat
3601 attgtttttt tatttggcat aactgtgatt cttttaggac aattactgta cacattaagg
3661 tgtatgtcag atattcatat tgacccaaat gtgtaatatt ccagttttct ctgcataagt
3721 aattaaaata tacttaaaaa ttaatagttt tatctgggta caaataaaca ggtgcctgaa
3781 ctagttcaca gacaaggaaa cttctatgta aaaatcacta tgatttctga attgctatgt
3841 gaaactacag atctttggaa cactgtttag gtagggtgtt aagacttaca cagtacctcg
3901 tttctacaca gagaaagaaa tggccatact tcaggaactg cagtgcttat gaggggatat
3961 ttaggcctct tgaatttttg atgtagatgg gcattttttt aaggtagtgg ttaattacct
4021 ttatgtgaac tttgaatggt ttaacaaaag atttgttttt gtagagattt taaaggggga
4081 gaattctaga aataaatgtt acctaattat tacagcctta aagacaaaaa tccttgttga
4141 agttttttta aaaaaagcta aattacatag acttaggcat taacatgttt gtggaagaat
4201 atagcagacg tatattgtat catttgagtg aatgttccca agtaggcatt ctaggctcta
4261 tttaactgag tcacactgca taggaattta gaacctaact tttataggtt atcaaaactg

4321 ttgtcaccat tgcacaattt tgtcctaata tatacataga aactttgtgg ggcatgttaa
4381 gttacagttt gcacaagttc atctcatttg tattccattg attttttttt tcttctaaac
4441 attttttctt caaacagtat ataacttttt ttaggggatt tttttttaga cagcaaaaac
4501 tatctgaaga tttccatttg tcaaaaagta atgatttctt gataattgtg tagtaatgtt
4561 ttttagaacc cagcagttac cttaaagctg aatttatatt tagtaacttc tgtgttaata
4621 ctggatagca tgaattctgc attgagaaac tgaatagctg tcataaaatg aaactttctt
4681 tctaaagaaa gatactcaca tgagttcttg aagaatagtc ataactagat taagatctgt
4741 gttttagttt aatagtttga agtgcctgtt tgggataatg ataggtaatt tagatgaatt
4801 taggggaaaa aaaagttatc tgcagatatg ttgagggccc atctctcccc ccacaccccc
4861 acagagctaa ctgggttaca gtgttttatc cgaaagtttc caattccact gtcttgtgtt
4921 ttcatgttga aaatactttt gcatttttcc tttgagtgcc aatttcttac tagtactatt
4981 tcttaatgta acatgtttac ctggaatgta ttttaactat ttttgtatag tgtaaactga
5041 aacatgcaca ttttgtacat tgtgctttct tttgtgggac atatgcagtg tgatccagtt
5101 gttttccatc atttggttgc gctgacctag gaatgttggt catatcaaac attaaaaatg
5161 accactcttt taattgaaat taacttttaa atgtttatag gagtatgtgc tgtgaagtga
5221 tctaaaattt gtaatatttt tgtcatgaac tgtactactc ctaattattg taatgtaata
5281 aaaatagtta cagtgacaaa aaaaaaaaaa aa

Human KRAS, transcript variant a, comprising the LCS6 SNP (KRAS-Variant), is encoded by the following mRNA sequence (SEQ ID NO: 11). (untranslated regions are bolded, LCS6 is underlined, SNP is capitalized):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gagagtggag gatgcttttt atacattggt
661 gagggagatc cgacaataca gattgaaaaa aatcagcaaa gaagaaaaga ctcctggctg
721 tgtgaaaatt aaaaaatgca ttataatgta atctgggtgt tgatgatgcc ttctatacat
781 tagttcgaga aattcgaaaa cataaagaaa agatgagcaa agatggtaaa aagaagaaaa
841 agaagtcaaa gacaaagtgt gtaattatgt aaatacaatt tgtacttttt tcttaaggca
901 tactagtaca agtggtaatt tttgtacatt acactaaatt attagcattt gttttagcat
961 tacctaattt ttttcctgct ccatgcagac tgttagcttt taccttaaat gcttatttta
1021 aaatgacagt ggaagttttt ttttcctcta agtgccagta ttcccagagt tttggttttt
1081 gaactagcaa tgcctgtgaa aaagaaactg aatacctaag atttctgtct tggggttttt
1141 ggtgcatgca gttgattact tcttattttt cttaccaatt gtgaatgttg gtgtgaaaca
1201 aattaatgaa gcttttgaat catccctatt ctgtgtttta tctagtcaca taaatggatt
1261 aattactaat ttcagttgag accttctaat tggtttttac tgaaacattg agggaacaca
1321 aatttatggg cttcctgatg atgattcttc taggcatcat gtcctatagt ttgtcatccc
1381 tgatgaatgt aaagttacac tgttcacaaa ggttttgtct cctttccact gctattagtc
1441 atggtcactc tccccaaaat attatatttt ttctataaaa agaaaaaaat ggaaaaaaat
1501 tacaaggcaa tggaaactat tataaggcca tttccttttc acattagata aattactata
1561 aagactccta atagcttttc ctgttaaggc agacccagta tgaaatgggg attattatag
1621 caaccatttt ggggctatat ttacatgcta ctaaattttt ataataattg aaaagatttt
1681 aacaagtata aaaaattctc ataggaatta aatgtagtct ccctgtgtca gactgctctt
1741 tcatagtata actttaaatc ttttcttcaa cttgagtctt tgaagatagt tttaattctg

1801 cttgtgacat taaaagatta tttgggccag ttatagctta ttaggtgttg aagagaccaa
1861 ggttgcaagg ccaggccctg tgtgaacctt tgagctttca tagagagttt cacagcatgg
1921 actgtgtccc cacggtcatc cagtgttgtc atgcattggt tagtcaaaat ggggagggac
1981 tagggcagtt tggatagctc aacaagatac aatctcactc tgtggtggtc ctgctgacaa
2041 atcaagagca ttgcttttgt ttcttaagaa aacaaactct tttttaaaaa ttacttttaa
2101 atattaactc aaaagttgag attttggggt ggtggtgtgc caagacatta attttttttt
2161 taaacaatga agtgaaaaag ttttacaatc tctaggtttg gctagttctc ttaacactgg
2221 ttaaattaac attgcataaa cacttttcaa gtctgatcca tatttaataa tgctttaaaa
2281 taaaaataaa aacaatcctt ttgataaatt taaaatgtta cttattttaa aataaatgaa
2341 gtgagatggc atggtgaggt gaaagtatca ctggactagg aagaaggtga cttaggttct
2401 agataggtgt cttttaggac tctgattttg aggacatcac ttactatcca tttcttcatg
2461 ttaaaagaag tcatctcaaa ctcttagttt ttttttttta caactatgta atttatattc
2521 catttacata aggatacact tatttgtcaa gctcagcaca atctgtaaat ttttaaccta
2581 tgttacacca tcttcagtgc cagtcttggg caaaattgtg caagaggtga agtttatatt
2641 tgaatatcca ttctcgtttt aggactcttc ttccatatta gtgtcatctt gcctccctac
2701 cttccacatg ccccatgact tgatgcagtt ttaatacttg taattcccct aaccataaga
2761 tttactgctg ctgtggatat ctccatgaag ttttcccact gagtcacatc agaaatgccc
2821 tacatcttat ttcctcaggg ctcaagagaa tctgacagat accataaagg gatttgacct
2881 aatcactaat tttcaggtgg tggctgatgc tttgaacatc tctttgctgc ccaatccatt
2941 agcgacagta ggatttttca aacctggtat gaatagacag aaccctatcc agtggaagga
3001 gaatttaata aagatagtgc tgaaagaatt ccttaggtaa tctataacta ggactactcc
3061 tggtaacagt aatacattcc attgttttag taaccagaaa tcttcatgca atgaaaaata
3121 ctttaattca tgaagcttac tttttttttt tggtgtcaga gtctcgctct tgtcacccag
3181 gctggaatgc agtggcgcca tctcagctca ctgcaacctc catctcccag gttcaagcga
3241 ttctcgtgcc tcggcctcct gagtagctgg gattacaggc gtgtgccact acactcaact
3301 aatttttgta tttttaggag agacggggtt tcaccctgtt ggccaggctg gtctcgaact
3361 cctgacctca agtgatGcac ccaccttggc ctcataaacc tgttttgcag aactcattta
3421 ttcagcaaat atttattgag tgcctaccag atgccagtca ccgcacaagg cactgggtat
3481 atggtatccc caaacaagag acataatccc ggtccttagg tagtgctagt gtggtctgta
3541 atatcttact aaggcctttg gtatacgacc cagagataac acgatgcgta ttttagtttt
3601 gcaaagaagg ggtttggtct ctgtgccagc tctataattg ttttgctacg attccactga
3661 aactcttcga tcaagctact ttatgtaaat cacttcattg ttttaaagga ataaacttga
3721 ttatattgtt tttttatttg gcataactgt gattctttta ggacaattac tgtacacatt
3781 aaggtgtatg tcagatattc atattgaccc aaatgtgtaa tattccagtt ttctctgcat
3841 aagtaattaa aatatactta aaaattaata gttttatctg ggtacaaata aacaggtgcc
3901 tgaactagtt cacagacaag gaaacttcta tgtaaaaatc actatgattt ctgaattgct
3961 atgtgaaact acagatcttt ggaacactgt ttaggtaggg tgttaagact tacacagtac
4021 ctcgtttcta cacagagaaa gaaatggcca tacttcagga actgcagtgc ttatgagggg
4081 atatttaggc ctcttgaatt tttgatgtag atgggcattt ttttaaggta gtggttaatt
4141 acctttatgt gaactttgaa tggtttaaca aaagatttgt ttttgtagag attttaaagg
4201 gggagaattc tagaaataaa tgttacctaa ttattacagc cttaaagaca aaaatccttg
4261 ttgaagtttt tttaaaaaaa gctaaattac atagacttag gcattaacat gtttgtggaa
4321 gaatatagca gacgtatatt gtatcatttg agtgaatgtt cccaagtagg cattctaggc
4381 tctatttaac tgagtcacac tgcataggaa tttagaacct aacttttata ggttatcaaa
4441 actgttgtca ccattgcaca attttgtcct aatatataca tagaaacttt gtggggcatg
4501 ttaagttaca gtttgcacaa gttcatctca tttgtattcc attgattttt tttttcttct
4561 aaacattttt tcttcaaaca gtatataact tttttagggg gatttttttt tagacagcaa
4621 aaactatctg aagatttcca tttgtcaaaa agtaatgatt tcttgataat tgtgtagtaa
4681 tgttttttag aacccagcag ttaccttaaa gctgaattta tatttagtaa cttctgtgtt
4741 aatactggat agcatgaatt ctgcattgag aaactgaata gctgtcataa aatgaaactt
4801 tctttctaaa gaaagatact cacatgagtt cttgaagaat agtcataact agattaagat
4861 ctgtgtttta gtttaatagt ttgaagtgcc tgtttgggat aatgataggt aatttagatg
4921 aatttagggg aaaaaaaagt tatctgcaga tatgttgagg gcccatctct cccccacac
4981 ccccacagag ctaactgggt tacagtgttt tatccgaaag tttccaattc cactgtcttg

5041 tgttttcatg ttgaaaatac ttttgcattt ttcctttgag tgccaatttc ttactagtac
5101 tatttcttaa tgtaacatgt ttacctggaa tgtattttaa ctatttttgt atagtgtaaa
5161 ctgaaacatg cacattttgt acattgtgct ttcttttgtg ggacatatgc agtgtgatcc
5221 agttgttttc catcatttgg ttgcgctgac ctaggaatgt tggtcatatc aaacattaaa
5281 aatgaccact cttttaattg aaattaactt ttaaatgttt ataggagtat gtgctgtgaa
5341 gtgatctaaa atttgtaata tttttgtcat gaactgtact actcctaatt attgtaatgt
5401 aataaaaata gttacagtga caaaaaaaaa aaaaaa

Human KRAS, transcript variant b, comprising the LCS6 SNP (KRAS-Variant), is encoded by the following mRNA sequence (SEQ ID NO: 12)(untranslated regions are bolded, LCS6 is underlined, SNP is capitalized):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gggtgttgat gatgccttct atacattagt
661 tcgagaaatt cgaaaacata aagaaaagat gagcaaagat ggtaaaaaga agaaaaagaa
721 gtcaaagaca aagtgtgtaa ttatgtaaat acaatttgta cttttttctt aaggcatact
781 agtacaagtg gtaatttttg tacattacac taaattatta gcatttgttt tagcattacc
841 taattttttt cctgctccat gcagactgtt agcttttacc ttaaatgctt attttaaaat
901 gacagtggaa gttttttttt cctctaagtg ccagtattcc cagagttttg gtttttgaac
961 tagcaatgcc tgtgaaaaag aaactgaata cctaagattt ctgtcttggg gtttttggtg
1021 catgcagttg attacttctt atttttctta ccaattgtga atgttggtgt gaaacaaatt
1081 aatgaagctt ttgaatcatc cctattctgt gttttatcta gtcacataaa tggattaatt
1141 actaatttca gttgagacct tctaattggt ttttactgaa acattgaggg aacacaaatt
1201 tatgggcttc ctgatgatga ttcttctagg catcatgtcc tatagtttgt catccctgat
1261 gaatgtaaag ttacactgtt cacaaaggtt ttgtctcctt tccactgcta ttagtcatgg
1321 tcactctccc caaaatatta tattttttct ataaaaagaa aaaaatggaa aaaaattaca
1381 aggcaatgga aactattata aggccatttc cttttcacat tagataaatt actataaaga
1441 ctcctaatag cttttcctgt taaggcagac ccagtatgaa atggggatta ttatagcaac
1501 cattttgggg ctatatttac atgctactaa atttttataa taattgaaaa gattttaaca
1561 agtataaaaa attctcatag gaattaaatg tagtctccct gtgtcagact gctctttcat
1621 agtataactt taaatctttt cttcaacttg agtctttgaa gatagtttta attctgcttg
1681 tgacattaaa agattatttg ggccagttat agcttattag gtgttgaaga gaccaaggtt
1741 gcaaggccag gccctgtgtg aacctttgag ctttcataga gagtttcaca gcatggactg
1801 tgtccccacg gtcatccagt gttgtcatgc attggttagt caaaatgggg agggactagg
1861 gcagtttgga tagctcaaca agatacaatc tcactctgtg gtggtcctgc tgacaaatca
1921 agagcattgc ttttgtttct taagaaaaca aactcttttt taaaaattac ttttaaatat
1981 taactcaaaa gttgagattt tggggtggtg gtgtgccaag acattaattt tttttttaaa
2041 caatgaagtg aaaaagtttt acaatctcta ggtttggcta gttctcttaa cactggttaa
2101 attaacattg cataaacact tttcaagtct gatccatatt taataatgct ttaaaataaa
2161 aataaaaaca atccttttga taaatttaaa atgttactta ttttaaaata aatgaagtga
2221 gatggcatgg tgaggtgaaa gtatcactgg actaggaaga aggtgactta ggttctagat
2281 aggtgtcttt taggactctg attttgagga catcacttac tatccatttc ttcatgttaa
2341 aagaagtcat ctcaaactct tagttttttt tttttacaac tatgtaattt atattccatt

2401 tacataagga tacacttatt tgtcaagctc agcacaatct gtaaattttt aacctatgtt
2461 acaccatctt cagtgccagt cttgggcaaa attgtgcaag aggtgaagtt tatatttgaa
2521 tatccattct cgttttagga ctcttcttcc atattagtgt catcttgcct ccctaccttc
2581 cacatgcccc atgacttgat gcagttttaa tacttgtaat tcccctaacc ataagattta
2641 ctgctgctgt ggatatctcc atgaagtttt cccactgagt cacatcagaa atgccctaca
2701 tcttatttcc tcagggctca agagaatctg acagatacca taaaggattt tgacctaatc
2761 actaatttcc aggtggtggc tgatgctttg aacatctctt tgctgcccaa tccattagcg
2821 acagtaggat ttttcaaacc tggtatgaat agacagaacc ctatccagtg gaaggagaat
2881 ttaataaaga tagtgctgaa agaattcctt aggtaatcta taactaggac tactcctggt
2941 aacagtaata cattccattg ttttagtaac cagaaatctt catgcaatga aaaatacttt
3001 aattcatgaa gcttactttt tttttttggt gtcagagtct cgctcttgtc acccaggctg
3061 gaatgcagtg gcgccatctc agctcactgc aacctccatc tcccaggttc aagcgattct
3121 cgtgcctcgg cctcctgagt agctgggatt acaggcgtgt gccactacac tcaactaatt
3181 tttgtatttt taggagagac ggggtttcac cctgttggcc aggctggtct cgaactcctg
3241 acctcaagtg atGcacccac cttggcctca taaacctgtt ttgcagaact catttattca
3301 gcaaatattt attgagtgcc taccagatgc cagtcaccgc acaaggcact gggtatatgg
3361 tatccccaaa caagagacat aatcccggtc cttaggtagt gctagtgtgg tctgtaatat
3421 cttactaagg cctttggtat acgacccaga gataacacga tgcgtatttt agttttgcaa
3481 agaaggggtt tggtctctgt gccagctcta taattgtttt gctacgattc cactgaaact
3541 cttcgatcaa gctactttat gtaaatcact tcattgtttt aaaggaataa acttgattat
3601 attgtttttt tatttggcat aactgtgatt cttttaggac aattactgta cacattaagg
3661 tgtatgtcag atattcatat tgacccaaat gtgtaatatt ccagttttct ctgcataagt
3721 aattaaaata tacttaaaaa ttaatagttt tatctgggta caaataaaca ggtgcctgaa
3781 ctagttcaca gacaaggaaa cttctatgta aaaatcacta tgatttctga attgctatgt
3841 gaaactacag atctttggaa cactgtttag gtagggtgtt aagacttaca cagtacctcg
3901 tttctacaca gagaaagaaa tggccatact tcaggaactg cagtgcttat gaggggatat
3961 ttaggcctct tgaatttttg atgtagatgg gcattttttt aaggtagtgg ttaattacct
4021 ttatgtgaac tttgaatggt ttaacaaaag atttgttttt gtagagattt taaaggggga
4081 gaattctaga aataaatgtt acctaattat tacagcctta aagacaaaaa tccttgttga
4141 agttttttta aaaaaagcta aattacatag acttaggcat taacatgttt gtggaagaat
4201 atagcagacg tatattgtat catttgagtg aatgttccca agtaggcatt ctaggctcta
4261 tttaactgag tcacactgca taggaattta gaacctaact tttataggtt atcaaaactg
4321 ttgtcaccat tgcacaattt tgtcctaata tatacataga aactttgtgg ggcatgttaa
4381 gttacagttt gcacaagttc atctcatttg tattccattg attttttttt tcttctaaac
4441 attttttctt caaacagtat ataacttttt ttaggggatt tttttttaga cagcaaaaac
4501 tatctgaaga tttccatttg tcaaaaagta atgatttctt gataattgtg tagtaatgtt
4561 ttttagaacc cagcagttac cttaaagctg aatttatatt tagtaacttc tgtgttaata
4621 ctggatagca tgaattctgc attgagaaac tgaatagctg tcataaaatg aaactttctt
4681 tctaaagaaa gatactcaca tgagttcttg aagaatagtc ataactagat taagatctgt
4741 gttttagttt aatagtttga agtgcctgtt tgggataatg ataggtaatt tagatgaatt
4801 taggggaaaa aaaagttatc tgcagatatg ttgagggccc atctctcccc ccacaccccc
4861 acagagctaa ctgggttaca gtgttttatc cgaaagtttc caattccact gtcttgtgtt
4921 ttcatgttga aaatactttt gcatttttcc tttgagtgcc aatttcttac tagtactatt
4981 tcttaatgta acatgtttac ctggaatgta ttttaactat ttttgtatag tgtaaactga
5041 aacatgcaca ttttgtacat tgtgctttct tttgtgggac atatgcagtg tgatccagtt
5101 gttttccatc atttggttgc gctgacctag gaatgttggt catatcaaac attaaaaatg
5161 accactcttt taattgaaat taacttttaa atgtttatag gagtatgtgc tgtgaagtga
5221 tctaaaattt gtaatatttt tgtcatgaac tgtactactc ctaattattg taatgtaata
5281 aaaatagtta cagtgacaaa aaaaaaaaaa aa

The present invention relates to a SNP within the 3'UTR of KRAS. Specifically, this SNP is the result of a substitution of a G for a U at position 4 of SEQ 10 NO: 6 of LCS6. This LCS6 SNP (KRAS-Variant) comprises the sequence GAUGCACCCACCUUGGCCUCA (SNP bolded for emphasis) (SEQ ID NO: 13).

The KRAS-Variant leads to altered *KRAS* expression by disrupting the miRNA regulation of a *KRAS.* The identification and characterization of the KRAS-Variant is further described in International Application No. PCT/US08/65302 (WO 2008/151004).

It was determined that the presence of the KRAS-Variant is associated with an increased risk of developing HBOS (or HBOC syndrome). The KRAS-Variant was found in approximately 22% of BRCA-positive and 61 % of BRCA-negative ovarian cancer patients from HBOC families. Interestingly, unlike BRCA mutations that are widely associated with HBOS (or HBOC syndrome) in individuals of Jewish descent, specifically, Ashkenazi descent, the KRAS-Variant does not appear to be primarily associated with individuals of Jewish descent. Thus, the KRAS-Variant provides a powerful tool for identifying HBOS (or HBOC syndrome) in BRCA-negative and non-Jewish individuals. Furthermore, the presence of both the KRAS-Variant and a BRCA1 mutation results in an increased the risk of that patient developing both breast and ovarian cancers. Over 60% of the individuals in the study who had both ovarian and breast cancer had both the KRAS-Variant and a BRCA1 mutation.

Accordingly, the invention features methods of predicting an increased risk of developing hereditary breast/ovarian cancer syndrome (HBOS or HBOC syndrome) in a subject, including detecting a single nucleotide polymorphism (SNP) at position 4 of the *let-7* complementary site 6 of KRAS in a patient sample. , wherein said subject is BRCA1 or BRCA2 mutation positive. The presence of the SNP indicates an increased risk of HBOS (or HBOC syndrome) in the subject.

In one aspect, the disclosure provides methods of identifying SNPs which increase the risk, susceptibility, or probability of developing a HBOS (HBOC syndrome). For example, a subject's risk of developing HBOS (HBOC syndrome), is determined by detecting a mutation in the 3' untranslated region (UTR) of a member of the KRAS gene superfamily. Specifically the mutation that is detected is a SNP at position 4 of LCS6 of KRAS of which results in a uracil (U) or thymine (T) to guanine (G) conversion.

The disclosure also features methods of predicting an increased risk of developing ovarian cancer or breast cancer in a subject, including detecting a BRCA1 mutation and a single nucleotide polymorphism (SNP) at position 4 of the *let-7* complementary site 6 of KRAS in a patient sample wherein the presence of the BRCA1 mutation and the SNP indicates an increased risk developing breast or ovarian cancer.

The disclosure further features methods of predicting an increased risk of developing both breast and ovarian cancer in a subject having HBOS (or HBOC syndrome) including detecting a BRCA1 mutation and a single nucleotide polymorphism (SNP) at position 4 of the *let-7* complementary site 6 of KRAS in a patient sample wherein the presence of the BRCA1 mutation and the SNP indicates an increased risk developing both breast and ovarian cancer.

Identification of the mutation indicates an increases risk of developing HBOS (or HBOC syndrome). "Risk" in the context of the present invention and disclosure, relates to the probability that an event will occur over a specific time period, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/ (1-p) where p is the probability of event and (1- p) is the probability of no event) to no-conversion.

"Risk evaluation," or "evaluation of risk" in the context of the present invention and disclosure encompasses making a prediction of the probability, odds, or likelihood that an event or disease state may occur, the rate of occurrence of the event or conversion from one disease state to another, i.e., from a primary tumor to a metastatic tumor or to one at risk of developing a metastatic, or from at risk of a primary metastatic event to a secondary metastatic event or from at risk of a developing a primary tumor of one type to developing a one or more primary tumors of a different type. Risk evaluation can also comprise prediction of future clinical parameters, traditional laboratory risk factor values, or other indices of cancer, either in absolute or relative terms in reference to a previously measured population.

An "increased risk" is meant to describe an increased probably that an individual who carries the KRAS-Variant, has HBOS (HBOC syndrome) and develop breast or ovarian cancer, compared to an individual who does not carry KRAS-Variant. In certain embodiments, a KRAS-Variant carrier is 1.5X, 2X, 2.5X, 3X, 3.5X, 4X, 4.5X, 5X, 5.5X, 6X, 6.5X, 7X, 7.5X, 8X, 8.5X, 9X, 9.5X, 10X, 20X, 30X, 40X, 50X, 60X, 70X, 80X, 90X, or 100X more likely to have HBOS (or HBOC syndrome) and develop breast or ovarian cancer than an individual who does not carry the KRAS-Variant.

By poor prognosis is meant that the probability of the individual surviving the development of particularly aggressive or high-risk subtypes of cancer is less than the probability of surviving more benign forms. Poor prognosis is also meant to describe a less satisfactory recovery, longer recovery period, more invasive or high-risk therapeutic regime, or an increased probability of reoccurrence of the cancer. It has been shown that the KRAS-Variant is predicative of the occurrence of aggressive subtypes of cancer. These aggressive subtypes of cancers are associated with the worst prognosis of each of these cancers resulting in a poor prognosis.

A subject is a human. A subject can be male or female. A subject is one who has not been previously diagnosed as having HBOS (or HBOC syndrome). The subject can be one who exhibits one or more risk factors for HBOS (or HBOC syndrome). Alternatively, the subject does not exhibit a risk factor for HBOS (or HBOC syndrome). HBOS (or HBOC syndrome) risk factors include for example, a parent or sibling who has been diagnosed with breast cancer, ovarian cancer, or both; a parent or sibling who has been diagnosed with pre-menopausal breast cancer; and Ashkenazi Jewish ancestry. The subject is BRCA-1 and/or BRCA-2 negative. Alternatively, the subject is BRCA-1 and/or BRCA-2 positive. In certain aspects, subjects are carriers of non-founder BRCA1 mutations. The subject is of Jewish descent. For example, the subject is of Ashkenazi Jewish descent. Alternatively, the subject is not of Jewish descent.

The biological sample can be any tissue or fluid that contains nucleic acids. Various embodiments include paraffin imbedded tissue, frozen tissue, surgical fine needle aspirations, and cells of the breast, endometrium, ovaries, uterus, or cervix. Other embodiments include fluid samples such peripheral blood lymphocytes, lymph fluid, ascites, serous fluid, sputum, and stool or urinary specimens such as bladder washing and urine.

Linkage disequilibrium (LD) refers to the co-inheritance of alleles (e.g., alternative nucleotides) at two or more different SNP sites at frequencies greater than would be expected from the separate frequencies of occurrence of each allele in a given population. The expected frequency of co-occurrence of two alleles that are inherited independently is the frequency of the first allele multiplied by the frequency of the second allele. Alleles that co-occur at expected frequencies are said to be in "linkage equilibrium". In contrast, LD refers to any non-random genetic association between allele(s) at two or more different SNP sites, which is generally due to the physical proximity of the two loci along a chromosome. LD can occur when two or more SNPs sites are in close physical proximity to each other on a given chromosome and therefore alleles at these SNP sites will tend to remain unseparated for multiple generations with the consequence that a particular nucleotide (allele) at one SNP site will show a non-random association with a particular nucleotide (allele) at a different SNP site located nearby. Hence, genotyping one of the SNP sites will give almost the same information as genotyping the other SNP site that is in LD.

For screening individuals for genetic disorders (e.g. prognostic or risk) purposes, if a particular SNP site is found to be useful for screening a disorder, then the skilled artisan would recognize that other SNP sites which are in LD with this SNP site would also be useful for screening the condition. Various degrees of LD can be encountered between two or more SNPs with the result being that some SNPs are more closely associated (i.e., in stronger LD) than others. Furthermore, the physical distance over which LD extends along a chromosome differs between different regions of the genome, and therefore the degree of physical separation between two or more SNP sites necessary for LD to occur can differ between different regions of the genome.

For screening applications, polymorphisms (e.g., SNPs and/or haplotypes) that are not the actual disease-causing (causative) polymorphisms, but are in LD with such causative polymorphisms, are also useful. In such instances, the genotype of the polymorphism(s) that is/are in LD with the causative polymorphism is predictive of the genotype of the causative polymorphism and, consequently, predictive of the phenotype (e.g., disease) that is influenced by the causative SNP(s). Thus, polymorphic markers that are in LD with causative polymorphisms are useful as markers, and are particularly useful when the actual causative polymorphism(s) is/are unknown.

Linkage disequilibrium in the human genome is reviewed in: Wall et al., "Haplotype blocks and linkage disequilibrium in the human genome", Nat Rev Genet. 2003 August; 4(8):587-97; Gamer et al., "On selecting markers for association studies: patterns of linkage disequilibrium between two and three diallelic loci", Genet Epidemiol. 2003 January; 24(1):57-67; Ardlie et al., "Patterns of linkage disequilibrium in the human genome", Nat Rev Genet. 2002 April; 3(4):299-309 (erratum in Nat Rev Genet 2002 July; 3(7):566); and Remm et al., "High-density genotyping and linkage disequilibrium in the human genome using chromosome 22 as a model"; Curr Opin Chem Biol. 2002 February; 6(1):24-30.

The screening techniques of the present invention and disclosure may employ a variety of methodologies to determine whether a test subject has a SNP or a SNP pattern associated with an increased or decreased risk of developing a detectable trait or whether the individual suffers from a detectable trait as a result of a particular polymorphism/mutation, including, for example, methods which enable the analysis of individual chromosomes for haplotyping, family studies, single sperm DNA analysis, or somatic hybrids. The trait analyzed using the diagnostics of the disclosure be any detectable trait that is commonly observed in pathologies and disorders.

### SNP Genotyping Methods

The process of determining which specific nucleotide (i.e., allele) is present at each of one or more SNP positions, such as a SNP position in a nucleic acid molecule disclosed in SEQ ID NO: 11, 12 or 13, is referred to as SNP genotyping. The present disclosure provides methods of SNP genotyping, such as for use in screening for a variety of disorders, or determining predisposition thereto, or determining responsiveness to a form of treatment, or prognosis, or in genome mapping or SNP association analysis, etc.

Nucleic acid samples can be genotyped to determine which allele(s) is/are present at any given genetic region (e.g., SNP position) of interest by methods well known in the art. The neighboring sequence can be used to design SNP detection reagents such as oligonucleotide probes, which may optionally be implemented in a kit format. Exemplary SNP genotyping methods are described in Chen et al., "Single nucleotide polymorphism genotyping: biochemistry, protocol, cost and throughput", Pharmacogenomics J. 2003; 3(2):77-96; Kwok et al., "Detection of single nucleotide polymorphisms", Curr Issues Mol. Biol. 2003 April; 5(2):43-60; Shi, "Technologies for individual genotyping: detection of genetic polymorphisms in drug targets and disease genes", Am J Pharmacogenomics. 2002; 2(3):197-205; and Kwok, "Methods for genotyping single nucleotide polymorphisms", Annu Rev Genomics Hum Genet 2001; 2:235-58. Exemplary techniques for high-throughput SNP genotyping are described in Marnellos, "High-throughput SNP analysis for genetic association studies", Curr Opin Drug Discov Devel. 2003 May; 6(3):317-21. Common SNP genotyping methods include, but are not limited to, TaqMan assays, molecular beacon assays, nucleic acid arrays, allele-specific primer extension, allele-specific PCR, arrayed primer extension, homogeneous primer extension assays, primer extension with detection by mass spectrometry, pyrosequencing, multiplex primer extension sorted on genetic arrays, ligation with rolling circle amplification, homogeneous ligation, OLA (U.S. Pat. No. 4,988,167), multiplex ligation reaction sorted on genetic arrays, restriction-fragment length polymorphism, single base extension-tag assays, and the Invader assay. Such methods may be used in combination with detection mechanisms such as, for example, luminescence or chemiluminescence detection, fluorescence detection, time-resolved fluorescence detection, fluorescence resonance energy transfer, fluorescence polarization, mass spectrometry, and electrical detection.

Various methods for detecting polymorphisms include, but are not limited to, methods in which protection from cleavage agents is used to detect mismatched bases in RNA/RNA or RNA/DNA duplexes (Myers et al., Science 230:1242 (1985); Cotton et al., PNAS 85:4397 (1988); and Saleeba et al., Meth. Enzymol. 217:286-295 (1992)), comparison of the electrophoretic mobility of variant and wild type nucleic acid molecules (Orita et al., PNAS 86:2766 (1989); Cotton et al., Mutat. Res. 285:125-144 (1993); and Hayashi et al., Genet. Anal. Tech. Appl. 9:73-79 (1992)), and assaying the movement of polymorphic or wild-type fragments in polyacrylamide gels containing a gradient of denaturant using denaturing gradient gel electrophoresis (DGGE) (Myers et al., Nature 313:495 (1985)). Sequence variations at specific locations can also be assessed by nuclease protection assays such as RNase and SI protection or chemical cleavage methods.

In a preferred embodiment, SNP genotyping is performed using the TaqMan assay, which is also known as the 5' nuclease assay (U.S. Pat. Nos. 5,210,015 and 5,538,848). The TaqMan assay detects the accumulation of a specific amplified product during PCR. The TaqMan assay utilizes an oligonucleotide probe labeled with a fluorescent reporter dye and a quencher dye. The reporter dye is excited by irradiation at an appropriate wavelength, it transfers energy to the quencher dye in the same probe via a process called fluorescence resonance energy transfer (FRET). When attached to the probe, the excited reporter dye does not emit a signal. The proximity of the quencher dye to the reporter dye in the intact probe maintains a reduced fluorescence for the reporter. The reporter dye and quencher dye may be at the 5' most and the 3' most ends, respectively, or vice versa. Alternatively, the reporter dye may be at the 5' or 3' most end while the quencher dye is attached to an internal nucleotide, or vice versa. In yet another embodiment, both the reporter and the quencher may be attached to internal nucleotides at a distance from each other such that fluorescence of the reporter is reduced.

During PCR, the 5' nuclease activity of DNA polymerase cleaves the probe, thereby separating the reporter dye and the quencher dye and resulting in increased fluorescence of the reporter. Accumulation of PCR product is detected directly by monitoring the increase in fluorescence of the reporter dye. The DNA polymerase cleaves the probe between the reporter dye and the quencher dye only if the probe hybridizes to the target SNP-containing template which is amplified during PCR, and the probe is designed to hybridize to the target SNP site only if a particular SNP allele is present.

Preferred TaqMan primer and probe sequences can readily be determined using the SNP and associated nucleic acid sequence information provided herein. A number of computer programs, such as Primer Express (Applied Biosystems, Foster City, Calif.), can be used to rapidly obtain optimal primer/probe sets. It will be apparent to one of skill in the art that such primers and probes for detecting the SNPs of the present invention and disclosure are useful in prognostic assays for a variety of disorders including cancer, and can be readily incorporated into a kit format. The present disclosure also includes modifications of the Taqman assay well known in the art such as the use of Molecular Beacon probes (U.S. Pat. Nos. 5,118,801 and 5,312,728) and other variant formats (U.S. Pat. Nos. 5,866,336 and 6,117,635).

The identity of polymorphisms may also be determined using a mismatch detection technique, including but not limited to the RNase protection method using riboprobes (Winter et al., Proc. Natl. Acad Sci. USA 82:7575, 1985; Meyers et al., Science 230:1242, 1985) and proteins which recognize nucleotide mismatches, such as the E. coli mutS protein (Modrich, P. Ann. Rev. Genet. 25:229-253, 1991). Alternatively, variant alleles can be identified by single strand conformation polymorphism (SSCP) analysis (Orita et al., Genomics 5:874-879, 1989; Humphries et al., in Molecular Diagnosis of Genetic Diseases, R. Elles, ed., pp. 321-340, 1996) or denaturing gradient gel electrophoresis (DGGE) (Wartell et al., Nuci. Acids Res. 18:2699-2706, 1990; Sheffield et al., Proc. Natl. Acad. Sci. USA 86:232-236, 1989).

A polymerase-mediated primer extension method may also be used to identify the polymorphism(s). Several such methods have been described in the patent and scientific literature and include the "Genetic Bit Analysis" method (WO92/15712) and the ligase/polymerase mediated genetic bit analysis (U.S. Pat. No. 5,679,524). Related methods are disclosed in WO91/02087, WO90/09455, WO95/17676, U.S. Pat. Nos. 5,302,509, and 5,945,283. Extended primers containing a polymorphism may be detected by mass spectrometry as described in U.S. Pat. No. 5,605,798. Another primer extension method is allele-specific PCR (Ruano et al., Nucl. Acids Res. 17:8392, 1989; Ruano et al., Nucl. Acids Res. 19, 6877-6882, 1991; WO 93/22456; Turki et al., J Clin. Invest. 95:1635-1641, 1995). In addition, multiple polymorphic sites may be investigated by simultaneously amplifying multiple regions of the nucleic acid using sets of allele-specific primers as described in Wallace et al. (WO89/10414).

Another preferred method for genotyping the SNPs is the use of two oligonucleotide probes in an OLA (see, e.g., U.S. Pat. No. 4,988,617). In this method, one probe hybridizes to a segment of a target nucleic acid with its 3' most end aligned with the SNP site. A second probe hybridizes to an adjacent segment of the target nucleic acid molecule directly 3' to the first probe. The two juxtaposed probes hybridize to the target nucleic acid molecule, and are ligated in the presence of a linking agent such as a ligase if there is perfect complementarity between the 3' most nucleotide of the first probe with the SNP site. If there is a mismatch, ligation would not occur. After the reaction, the ligated probes are separated from the target nucleic acid molecule, and detected as indicators of the presence of a SNP.

The following patents, patent applications, and published international patent applications provide additional information pertaining to techniques for carrying out various types of OLA: U.S. Pat. Nos. 6,027,889, 6,268,148, 5494810, 5830711, and 6054564 describe OLA strategies for performing SNP detection; WO 97/31256 and WO 00/56927 describe OLA strategies for performing SNP detection using universal arrays, wherein a zipcode sequence can be introduced into one of the hybridization probes, and the resulting product, or amplified product, hybridized to a universal zip code array; U.S. application US01/17329 (and Ser. No. 09/584,905) describes OLA (or LDR) followed by PCR, wherein zipcodes are incorporated into OLA probes, and amplified PCR products are determined by electrophoretic or universal zipcode array readout; U.S. application 60/427,818, 60/445,636, and 60/445,494 describe SNPlex methods and software for multiplexed SNP detection using OLA followed by PCR, wherein zipcodes are incorporated into OLA probes, and amplified PCR products are hybridized with a zipchute reagent, and the identity of the SNP determined from electrophoretic readout of the zipchute. In some embodiments, OLA is carried out prior to PCR (or another method of nucleic acid amplification). In other embodiments, PCR (or another method of nucleic acid amplification) is carried out prior to OLA.

Another method for SNP genotyping is based on mass spectrometry. Mass spectrometry takes advantage of the unique mass of each of the four nucleotides of DNA. SNPs can be unambiguously genotyped by mass spectrometry by measuring the differences in the mass of nucleic acids having alternative SNP alleles. MALDI-TOF (Matrix Assisted Laser Desorption Ionization--Time of Flight) mass spectrometry technology is preferred for extremely precise determinations of molecular mass, such as SNPs. Numerous approaches to SNP analysis have been developed based on mass spectrometry. Preferred mass spectrometry-based methods of SNP genotyping include primer extension assays, which can also be utilized in combination with other approaches, such as traditional gel-based formats and microarrays.

Typically, the primer extension assay involves designing and annealing a primer to a template PCR amplicon upstream (5') from a target SNP position. A mix of dideoxynucleotide triphosphates (ddNTPs) and/or deoxynucleotide triphosphates (dNTPs) are added to a reaction mixture containing template (e.g., a SNP-containing nucleic acid molecule which has typically been amplified, such as by PCR), primer, and DNA polymerase. Extension of the primer terminates at the first position in the template where a nucleotide complementary to one of the ddNTPs in the mix occurs. The primer can be either immediately adjacent (i.e., the nucleotide at the 3' end of the primer hybridizes to the nucleotide next to the target SNP site) or two or more nucleotides removed from the SNP position. If the primer is several nucleotides removed from the target SNP position, the only limitation is that the template sequence between the 3' end of the primer and the SNP position cannot contain a nucleotide of the same type as the one to be detected, or this will cause premature termination of the extension primer. Alternatively, if all four ddNTPs alone, with no dNTPs, are added to the reaction mixture, the primer will always be extended by only one nucleotide, corresponding to the target SNP position. In this instance, primers are designed to bind one nucleotide upstream from the SNP position (i.e., the nucleotide at the 3' end of the primer hybridizes to the nucleotide that is immediately adjacent to the target SNP site on the 5' side of the target SNP site). Extension by only one nucleotide is preferable, as it minimizes the overall mass of the extended primer, thereby increasing the resolution of mass differences between alternative SNP nucleotides. Furthermore, mass-tagged ddNTPs can be employed in the primer extension reactions in place of unmodified ddNTPs. This increases the mass difference between primers extended with these ddNTPs, thereby providing increased sensitivity and accuracy, and is particularly useful for typing heterozygous base positions. Mass-tagging also alleviates the need for intensive sample-preparation procedures and decreases the necessary resolving power of the mass spectrometer.

The extended primers can then be purified and analyzed by MALDI-TOF mass spectrometry to determine the identity of the nucleotide present at the target SNP position. In one method of analysis, the products from the primer extension reaction are combined with light absorbing crystals that form a matrix. The matrix is then hit with an energy source such as a laser to ionize and desorb the nucleic acid molecules into the gas-phase. The ionized molecules are then ejected into a flight tube and accelerated down the tube towards a detector. The time between the ionization event, such as a laser pulse, and collision of the molecule with the detector is the time of flight of that molecule. The time of flight is precisely correlated with the mass-to-charge ratio (m/z) of the ionized molecule. Ions with smaller m/z travel down the tube faster than ions with larger m/z and therefore the lighter ions reach the detector before the heavier ions. The time-of-flight is then converted into a corresponding, and highly precise, m/z. In this manner, SNPs can be identified based on the slight differences in mass, and the corresponding time of flight differences, inherent in nucleic acid molecules having different nucleotides at a single base position. For further information regarding the use of primer extension assays in conjunction with MALDI-TOF mass spectrometry for SNP genotyping, see, e.g., Wise et al., "A standard protocol for single nucleotide primer extension in the human genome using matrix-assisted laser desorption/ionization time-of flight mass spectrometry", Rapid Commun Mass Spectrom. 2003; 17(11):1195-202.

The following references provide further information describing mass spectrometry-based methods for SNP genotyping: Bocker, "SNP and mutation discovery using base-specific cleavage and MALDI-TOF mass spectrometry", Bioinformatics. 2003 July; 19 Suppl 1:144-153; Storm et al., "MALDI-TOF mass spectrometry-based SNP genotyping", Methods Mol. Biol. 2003;212:241-62; Jurinke et al., "The use of MassARRAY technology for high throughput genotyping", Adv Biochem Eng Biotechnol. 2002;77:57-74; and Jurinke et al., "Automated genotyping using the DNA MassArray technology", Methods Mol. Biol. 2002;187:179-92.

SNPs can also be scored by direct DNA sequencing. A variety of automated sequencing procedures can be utilized ((1995) Biotechniques 19:448), including sequencing by mass spectrometry (see, e.g., PCT International Publication No. WO94/16101; Cohen et al., Adv. Chromatogr. 36:127-162 (1996); and Griffn et al., Appl. Biochem. Biotechnol. 38:147-159

(1993)). The nucleic acid sequences of the present disclosure enable one of ordinary skill in the art to readily design sequencing primers for such automated sequencing procedures. Commercial instrumentation, such as the Applied Biosystems 377, 3100, 3700, 3730, and 3730.times.1 DNA Analyzers (Foster City, Calif.), is commonly used in the art for automated sequencing.

Other methods that can be used to genotype the SNPs of the present disclosure include single-strand conformational polymorphism (SSCP), and denaturing gradient gel electrophoresis (DGGE) (Myers et al., Nature 313:495 (1985)). SSCP identifies base differences by alteration in electrophoretic migration of single stranded PCR products, as described in Orita et al., Proc. Nat. Acad. Single-stranded PCR products can be generated by heating or otherwise denaturing double stranded PCR products. Single-stranded nucleic acids may refold or form secondary structures that are partially dependent on the base sequence. The different electrophoretic mobilities of single-stranded amplification products are related to base-sequence differences at SNP positions. DGGE differentiates SNP alleles based on the different sequence-dependent stabilities and melting properties inherent in polymorphic DNA and the corresponding differences in electrophoretic migration patterns in a denaturing gradient gel (Erlich, ed., PCR Technology, Principles and Applications for DNA Amplification, W. H. Freeman and Co, New York, 1992, Chapter 7).

Sequence-specific ribozymes (U.S. Pat. No. 5,498,531) can also be used to score SNPs based on the development or loss of a ribozyme cleavage site. Perfectly matched sequences can be distinguished from mismatched sequences by nuclease cleavage digestion assays or by differences in melting temperature. If the SNP affects a restriction enzyme cleavage site, the SNP can be identified by alterations in restriction enzyme digestion patterns, and the corresponding changes in nucleic acid fragment lengths determined by gel electrophoresis

SNP genotyping can include the steps of, for example, collecting a biological sample from a human subject (e.g., sample of tissues, cells, fluids, secretions, etc.), isolating nucleic acids (e.g., genomic DNA, mRNA or both) from the cells of the sample, contacting the nucleic acids with one or more primers which specifically hybridize to a region of the isolated nucleic acid containing a target SNP under conditions such that hybridization and amplification of the target nucleic acid region occurs, and determining the nucleotide present at the SNP position of interest, or, in some assays, detecting the presence or absence of an amplification product (assays can be designed so that hybridization and/or amplification will only occur if a particular SNP allele is present or absent). In some assays, the size of the amplification product is detected and compared to the length of a control sample; for example, deletions and insertions can be detected by a change in size of the amplified product compared to a normal genotype.

### EXAMPLES

### Example 1: Prevalence of the KRAS-Variant

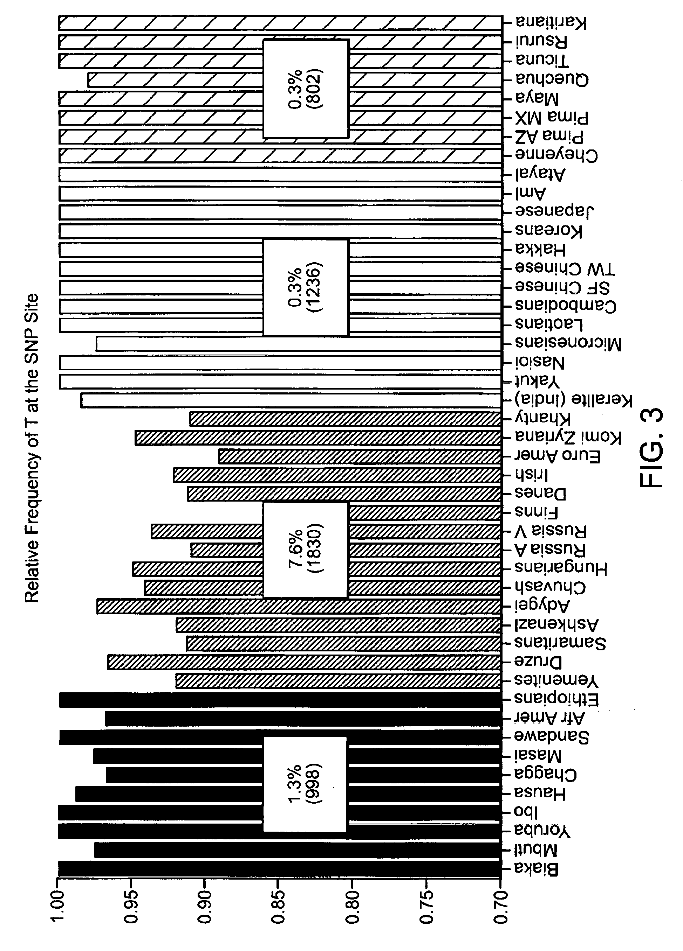

As shown in Figure 3, the prevalence of the KRAS-Variant, also referred to as the Onco-SNP, was evaluated within an ethnically diverse sample of 2500 subjects representing 46 geographic populations. The KRAS-Variant is more prevalent in the Caucasian population of the United States, at 11 %, than in the world's population (6 % average).

### Example 2: KRAS-Variant in Ovarian Cancer

The KRAS-Variant, is present in up to 27% of newly diagnosed ovarian cancer patients. Among patients of Northern Italian origin (providing 215 samples to the study), the KRAS-Variant was present in 25% of the samples provided. Thus, the positive predictive value within this population is 6%, which means that I of every 16 KRAS-Variant-positive individual will develop ovarian cancer.

Among patients treated at Yale University, the prevalence of the KRAS-Variant was approximately the same as in the Northern Italian patient group. Yale University patients provided 100 samples to the study. The KRAS-Variant was found in 36 % of those samples. Thus, the positive predictive value within the Yale University patient population is also 6%. Similar to the Northern Italian population, these results show that 1 of every 16 KRAS-Variant - positive individuals will develop ovarian cancer.

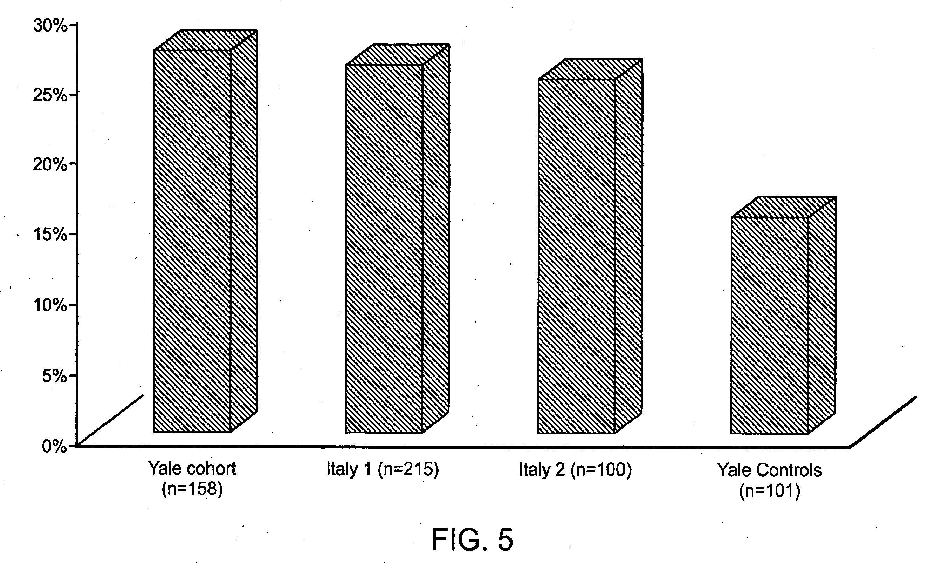

About 1/4 of all ovarian cancer patients have the KRAS-Variant (Figure 5).

### Example 3: KRAS-Variant Predicts an Increased Risk of Developing Ovarian Cancer

Comparisons of "wild-type" individuals and those individuals carrying the KRAS-Variant revealed that the presence of the KRAS-Variant is predictive of a 2.0-fold increased risk of developing ovarian cancer compared to those who do not carry either a single or double copy of the mutation. Comparisons were adjusted for both age and race.

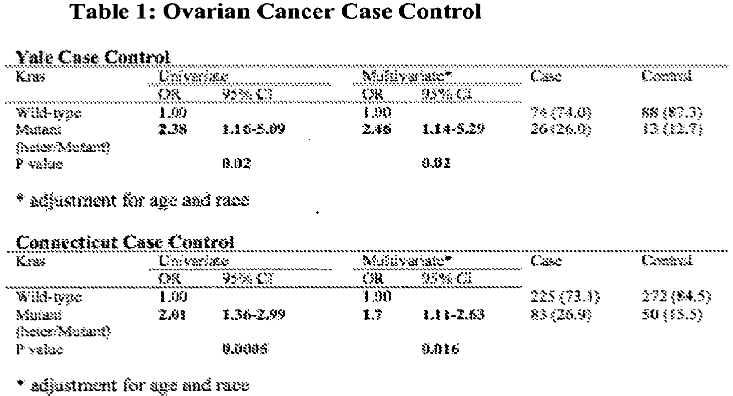

When age is considered, comparisons between wild type or normal individuals who do not carry the KRAS-Variant and those individuals who carry either a one or two copies of the KRAS-Variant reveal that the KRAS-Variant (or Onco-SNP) is as prevalent in older women as younger women (Table 2). Moreover, the prevalence of the KRAS-Variant in women of various ages does not vary by ovarian cancer subtype. The KRAS-Variant is not associated with cancer for younger women.

**Table 2: Age Comparison of Ovarian Cancer Prevalence**

| **Onco-SNP Genotype** | **u** | **Age** | |
|---|---|---|---|
| | | **Median** | **Range (Min-Max)** |
| **00** | 200 | 58.5 | 24.0-82.1 |
| **1/2** | 68 | 56.4 | 37.4-80.2 |
| **p value** | | 0.478 | |

### Example 4: Comparison of KRAS-Variant and non- KRAS-Variant Ovarian Cancer Patients

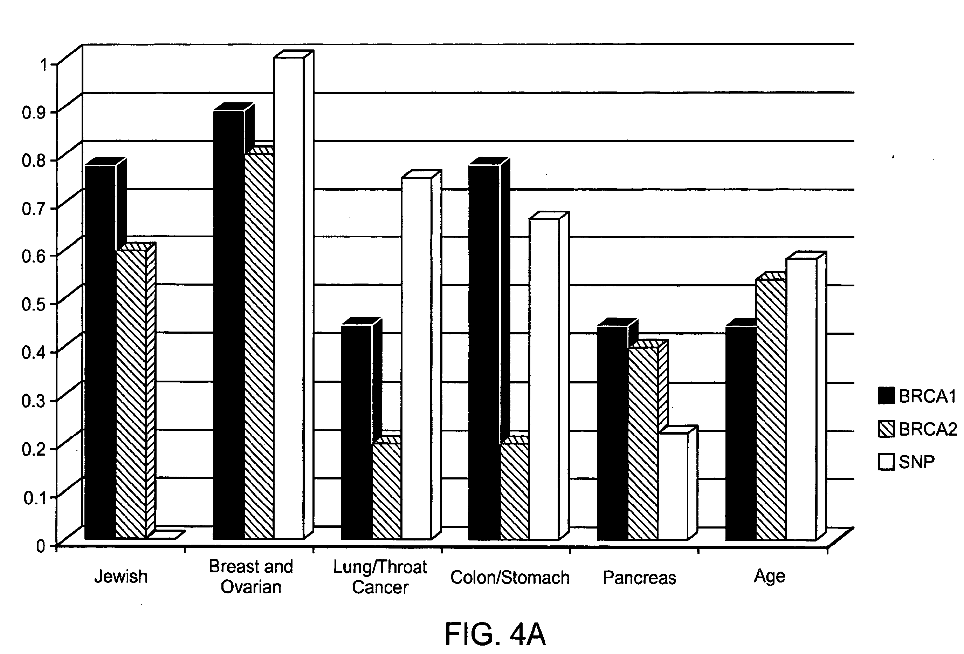

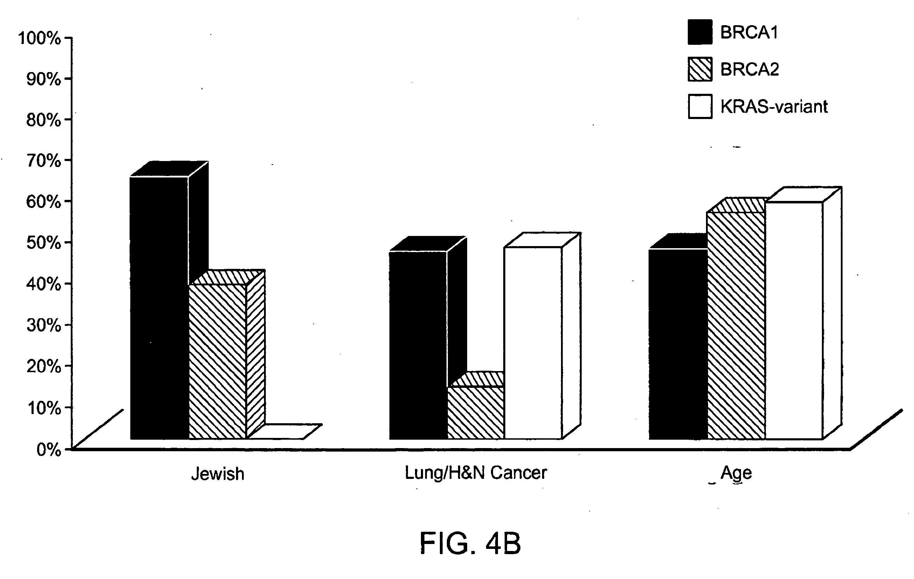

In a comparison of KRAS-Variant and non- KRAS-Variant ovarian cancer patients, it was determined that the median age of ovarian cancer onset for someone with the KRAS-Variant was younger than someone who did not have the KRAS-Variant (referred to as a non-KRAS-Variant patient). KRAS-Variant carriers presented ovarian cancer onset at an average age of 57.8 years versus 59 years of age for non-SNP patients (Figure 4 for age group and Table 2).

Moreover, results of ovarian subtype comparisons revealed significant differences in progression-free survival (PFS) or overall survival (OS). Table 3 shows an ovarian cancer subtype analysis quantifying the number (and percentage) of patients having each cancer type, broken down by KRAS-Variant status. The data show that the association of KRAS-Variant with non-mucinous ovarian cancer is significantly higher than in mucinous cancer (p = 0.031 which is less that 0.05).

**Table 3: Ovarian Cancer Subtype Analysis**

| **Histological Type** | **number** | **Non-onco-SNP** | **Onco-SNP** |
|---|---|---|---|
| CC/MU/OT | 33 | 21 (63.6) | 12 (36.4) |
| HN | 45 | 33 (73.3) | 12 (26.7) |
| MC | 20 | 19 (95.0) | 1 (5.0) |
| UN | 36 | 29 (80.6) | 7 (19.4) |
| SP | 129 | 95 (73.6) | 34 (26.4) |
| P value | | 0.121 | |

| **Histological Type 2** | | | |
|---|---|---|---|
| MC | 20 | 19 (95.0) | 1 (5.0) |
| Non-MC | 243 | 178 (73.3) | 65 (27.7) |
| P value | | **0.031** | |

| | | | |
|---|---|---|---|
| CC = clear cell, MU = mullerian, OT = other, EN = endometriod, MC = mucinous, UN = undifferentiated, and SP = papillary serious. | | | |

A corroborating study also demonstrated that the KRAS-Variant, or Onco-SNP is rare in ovarian cancer patients with mucinous tumors (Table 4).

**Table 4: Ovarian Cancer Subtype Comparison**

| **Subtypes** | ***n*** | **Non-onco-SNP patients (%)** | **onco-SNP patients (%)** |
|---|---|---|---|
| **CC** | **22** | **14 (63.6)** | **8 (36.4)** |
| **MU** | **15** | **10 (66.7)** | **5 (33.3)** |
| **EN** | **52** | **37 (71.2)** | **15 (28.9)** |
| **SP** | **167** | **127 (76.1)** | **40 (23.9)** |
| **UN** | **37** | **30 (81.1)** | **7 (18.9)** |
| **MC** | **22** | **20 (90.9)** | **2 (9.1)** |
| **p value** | | **0.394** | |

### Example 5: Association of KRAS-Variant with Hereditary Breast/Ovarian Cancer

Ovarian cancer patients provided four-generation pedigrees that included occurrences of both breast and ovarian cancer. Furthermore, the BRCA1 and BRCA2 status of each ovarian cancer patient included in the study was known. Among these study participants, 36 BRCA-positive ovarian cancer patients were assessed for the presence of the KRAS-Variant. Among these BRCA-positive individuals, 30% (or 23 individuals) were positive for both the BRCA1 and the KRAS-Variant mutations. Moreover, 8% (or 13 individuals) were positive for both the BRCA2 and the KRAS-Variant mutations. These same results were demonstrated when breast cancer patients were evaluated. Also, in this study, 31 BRCA-negative ovarian cancer patients were assessed for the presence of the KRAS-Variant. Among this BRCA-negative ovarian cancer population, 61% of the individuals were positive for the KRAS-Variant with a statistical significance of p < 0.0001. As a result of this study, the positive predictive value (PPV) of the KRAS-Variant mutation increased to 7.4 %. Thus, 1 of 12 people with the KRAS-Variant is at risk of developing ovarian cancer consistent with a family history of HBOC syndrome (or HBOS). The negative predictive value (NPV) of the KRAS-Variant mutation was 99.4% (1/167 risk). Individuals who carry the KRAS-Variant are 6x more likely to develop ovarian cancer.

The data show that the presence or absence of the KRAS-Variant is a more reliable predictor of the risk of developing hereditary breast/ovarian cancer (HBOC) than BRCA. BRCA is only effective as a predictive marker, rather than a risk factor, for about 5% of patients, who are usually of Ashkenazi, Eastern European, Jewish backgrounds. Genetic tests for the presence of KRAS-Variant, either alone, or in combination with BRCA, are used to predict the risk of breast and/or ovarian cancer in any patient. The KRAS-Variant test is particularly valuable for those patients who are BRCA-negative, and for whom, until now, no test has existed. The KRAS-Variant test is not only a valuable initial screening tool, because of the simplicity of the test, compared to BRCA for instance, but the KRAS-Variant test is of particular value for BRCA-negative and HBOS (or HBOC syndrome) individuals. An HBOS (or HBOC syndrome) individual is someone who has either themselves been diagnosed with Hereditary Breast/Ovarian Syndrome (HBOS or HBOC syndrome), or is related to someone diagnosed with HBOS (or HBOC syndrome).

Importantly, the data show that while BRCA and KRAS-Variant are individually predictive of cancer risk, the occurrence of both mutations in the same individual has an additive, and probably a synergistic effect. For instance, of the co-positive patients in this study, approximately 65% had both ovarian and breast.cancer. Individually, the KRAS-Variant occurs in one third of ovarian cancer cases. The BRCA1 mutation occurred in 34% of the ovarian cancer patients of this study. Of the 30% of ovarian cancer patient who were co-positive for BRCA1 and KRAS-Variant, a resounding 65 % had developed both cancers.

BRCA-positive patients represent a small proportion of the population, approximately less than 10 percent of ovarian cancer patients. In contrast, the KRAS-Variant occurs in 36 percent of ovarian cancer patients.

Similar results were demonstrated in breast cancer BRCA-positive cohort of patients. 29% of 129 BRCA1 positive breast cancer patients were co-positive for the KRAS-variant, whereas 11% of 156 BRCA2 positive breast cancer patients were co-positive for the KRAS-variant.

### Example 6: Association of the KRAS-Variant with Hereditary Breast/Ovarian Cancer

The relative prevalence of the BRCA 1, BRCA2, and KRAS-Variant mutations were assessed within various ethnic, diagnostic, and age groups. Figure 4 shows that while BRCA1 and BRCA2 more effective predictors of cancer risk among Jewish patients, of Eastern European descent, the KRAS-Variant is a more reliable marker of breast/ovarian and lung/throat cancer than either BRCA1 or BRCA2. With respect to colon and stomach cancer, the KRAS-Variant is more prevalent than BRCA1. Similar to the breast/ovarian and lung/throat groups, the KRAS-Variant is the most prevalent marker of cancer with increasing age. Although individuals with HBOS (or HBOC syndrome) are often diagnosed at an early age, the KRAS-Variant is a predictor of cancer onset in patients with advancing age. This quality of the KRAS-Variant test is further increased by the ability of this mutation to predict the increased risk of cancer onset in a patient population that has not yet been recognized.

The data of Figure 4 elucidate several target patient populations who would most benefit from diagnostic or prognostic testing for the KRAS-Variant. Among cancer patients, those who have a family history, a sign, a symptom, a risk factor, or a diagnosis of breast, ovarian, lung, or throat cancer. As stated above, cancer patients of advanced age would particularly benefit from testing for the KRAS-Variant. Importantly, these results show that the BRCA-negative population is a specific target for KRAS-Variant testing because the presence of this mutation is associated with one third of ovarian cancer patients and 63% of non-BRCA HBOC families. The KRAS-Variant is also predictive of a risk of developing ovarian cancer of up to 1/11. Families affected by the KRAS-Variant are significantly more likely to be non-Jewish and to experience later onset cancers.

### Example 7: The KRAS-Variant Predicts Ovarian Cancer Aggressiveness and Response to Treatment

Comparisons of individuals who do not carry the KRAS-Variant (or the Onco SNP), labeled as "00", and those individuals who carry one or two copies of the KRAS-Variant, "1/2," revealed that the presence of the KRAS-Variant is associated with more advanced cancer, which is classified as stage III-IV (Table 5). Moreover, the KRAS-Variant is also associated with more aggressive ovarian cancer, which is non-responsive or less responsive to known treatments (Table 5).

**[87] Table 5: Ovarian Cancer Aggressiveness**

| **Variables** | ***n*** | **Onco-SNP Genotype** | |
|---|---|---|---|
| | | **00** | **1/2** |
| **Disease stage** | | | |
| I-II | 64 | 51 (79.7) | 13 (20.3) |
| III-IV | 147 | 106(72.1) | 41 (27.9) |
| p value | | 0.246 | |

| **Variables** | ***n*** | **Onco-SNP Genotype** | |
|---|---|---|---|
| | | **00** | **1/2** |
| **Treatment response** | | | |
| No | 74 | 52 (70.3) | 22 (29.7) |
| Yes | 183 | 139 (76.0) | 44 (24.0) |
| p value | | 0.345 | |

### Example 8: The KRAS-Variant Predicts Poor Prognosis for Ovarian Cancer Patients

Comparisons of individuals who do not carry the KRAS-Variant, labeled as "00", and those individuals who carry one or two copies of the KRAS-Variant, "Variant (heter/homoz)," revealed that the presence of the KRAS-Variant is associated with more poor prognosis, which is reflects poor survival or increased rates of patient death (Table 6). The multivariate comparisons were adjusted for age and ethnicity.

**Table 6: Ovarian Cancer Disease Outcome**

| *KRAS All (n = 598)* | Univariate | | Multivariate* | |
|---|---|---|---|---|
| | OR | 95% Cl | OR | 95% Cl |
| Wild-type | 1.00 | | 1.00 | |
| Variant (heter/homoz) | 1.09 | 0.84-1.42 | 1.1 | 0.85-1.44 |
| | | | | |

| *KRAS >60 (n = 246)* | Univariate | | Multivariate* | |
|---|---|---|---|---|
| | OR | 95% Cl | OR | 95% Cl |
| Wild-type | 1.00 | | 1.00 | |
| Variant (heter/homoz) | **1.44** | 1.00-2.07 | **1.45** | 1.01-2.08 |
| | | | | |

| *KRAS >60 and Cauc (n* = *243)* | Univariate | | Multivariate* | |
|---|---|---|---|---|
| | OR | 95% Cl | OR | 95% Cl |
| Wild-type | 1.00 | | 1.00 | |
| Variant (heter/homoz) | **1.49** | 1.03-2.14 | **1.47** | 1.02-2.12 |

| | | | | |
|---|---|---|---|---|
| *Adjustment for age and ethnicity | | | | |

### Example 9: The KRAS-Variant is a Genetic Marker of Hereditary Breast and Ovarian Cancer Syndrome.

The KRAS-Variant is associated with ovarian cancer risk for sporadic ovarian cancer. To further validate the role of the KRAS-Variant as a genetic marker of ovarian cancer, those ovarian cancer patients who were considered to be at high-risk for having a familial genetic abnormality with a family history consistent with Hereditary Breast and Ovarian Cancer (HBOC) Syndrome were further examined for the presence of the KRAS-Variant. These patients had either a personal and/or family history (within 1st or 2nd degree relatives) of at least one additional case of ovarian cancer and/or breast cancer. Moreover, all of the patients included in this study were of European ancestry. All of the study participants had also undergone BRCA mutation analysis. Sixty-seven patients fit the following parameters: 23 were positive for BRCA1 mutations; 13 were positive for BRCA2 mutations; and 31 were uninformative (negative for both BRCA1 and BRCA2 mutations). Overall, 8/36 (or 22%) of BRCA mutation carriers were carriers for the KRAS-Variant. Specifically, 7/23 (or 30%) of BRCA1 mutant carriers were co-positive for the KRAS-Variant and 1/13 (or 8%) of BRCA2 mutant carriers were co-positive for the KRAS-Variant. The differential association of the KRAS-Variant with BRCA1 and BRCA2 represents a biological modification of BRCA penetrance by the KRAS-Variant.

Enhancement of BRCA1 by the KRAS-Variant was tested in a larger cohort of breast cancer patients. Of the 300 breast cancer patients that were BRCA1 and BRCA2 positive, 150 had BRCA1 mutations, and 150 had BRCA2 mutations. Similar to the ovarian cancer study, the KRAS-Variant was present in 30% breast cancer patients with the BRCA1 mutation, and in only 10% of breast cancer patients with the BRCA2 mutation. These results confirm our hypothesis that there is an enhanced risk of developing either breast or ovarian cancer for individuals who carry both BRCA1 and the KRAS-Variant.

Segregation analysis is ongoing. The results are expected to reveal an increased risk of developing breast and/or ovarian cancer for individuals who carry the BRCA1 and the KRAS-Variant mutations. The data have demonstrated that an individual is significantly more likely to develop breast or ovarian cancer when she carries a BRCA1 mutation and the KRAS-Variant. Thus, the KRAS-Variant modifies BRCA1 penetrance. In fact, the KRAS-Variant is one of the strongest known modifiers of BRCA1 penetrance.

### Example 10: The KRAS-Variant is a Genetic Marker of Ovarian Cancer Risk

Ovarian cancer is the single most deadly form of womens cancer, largely due to patients presenting with advanced disease due to a lack of known risk factors or genetic markers of risk. The KRAS oncogene and altered levels of the microRNA let-7 are associated with an increased risk of developing solid tumors. The association of the variant (derived) allele at rs61764370, referred to as the KRAS-Variant, previously shown to disrupt a let-7 microRNA binding site in the KRAS oncogene, was investigated and demonstrated increased ovarian cancer risk.

Specimens were obtained and tested for the presence of the KR.AS-Variant from non-selected ovarian cancer patients in three independent cohorts (n = 472), from two independent ovarian casecontrol studies (n = 866), and from ovarian. cancer patients with hereditary breast and ovarian cancer (HBOC) syndrome (n = 67) as well as in their family members.

The results indicate that the KRAS-variant is associated with greater than 25% of non-selected ovarian cancer cases, and is a marker for a significant increased risk of developing ovarian cancer as confirmed by two independent case control analyses. In addition, the KRAS-variant was identified in 61% of HBOC patients without BRCA1 or BRCA2 mutations, previously considered uninformative, as well as in their family members with cancer. These findings strongly suggest that the KRAS-variant is a genetic marker of an increased risk of developing ovarian cancer, and further suggests that the KRAS-variant is a new genetic marker of cancer risk for HBOC families without other known genetic abnormalities.

### The KRAS-variant and Ovarian Cancer Risk

Women with epithelial ovarian cancer (OC) who presented at Yale/New Haven Hospital for surgery (n = 157) were tested for the KRAS-variant. It was discovered that over 27% of these women harbored this variant-allele. Because this was a significantly higher prevalence than previously shown in any normal or cancerous population (18%, 14, 22 and > 9,000 additional people tested), this finding was validated in two additional, independent cohorts of epithelial OC patients. The first was from the University Hospital in Northern Italy at the University of Turin (*n* = 215), and 26% of patients harbored the *KRAS*-variant in this cohort. The second was from Brescia, Italy (*n* = 100), and again 25% of these OC patients carried the *KRAS-*variant. The frequency of the KRAS-variant was thus significantly higher in these OC cohorts than in any group previously studied, including non-cancerous controls collected at Yale New Haven Hospital (Figure 5).

To investigate if the *KRAS*-variant predicts an increased risk of developing OC for non-selected female populations, case-control analyses were performed. The Yale Case-Control contained 100 cases and 101 controls and showed a significantly increased risk of developing OC for *KRAS*-variant carriers by multivariate analysis (OR = 2.46, CI = 1.14-5.29, p = 0.020). These findings were validated in a second independent case control: the Connecticut Ovarian Cancer Case-Control consists of 320 patients and 328 controls, and also showed a significant increased risk of developing OC for the *KRAS*-variant carriers by multivariate analysis (OR = 1.7, CI = 1.11-2.63, p = 0.016) (Table 1). These findings suggest that the *KRAS*-variant is a genetic marker of an increased risk of developing OC in non-selected women. *Ovarian Cancer Variables and the KRAS-variant*

The distribution of the *KRAS*-variant was evaluated in the different subtypes of epithelial OC. It was found that the prevalence of the *KRAS*-variant varied between subtypes, being most common in non-mucinous cancers, but was rarely found in patients with mucinous ovarian cancers (p<0.05, Table 4).

A range of variables were studied to determine if there were specific characteristics segregating OC patients harboring the *KRAS*-variant versus those without. It was found that in patients with available data there was not a significant difference in patient age at first surgery, tumor grade, residual tumor size, debulking, stage of OC presentation, response to platinum-based chemotherapy, or progression free survival (hazard ratio (HR)= 1.12, 95% CI 0.71-1.76) (Table 7).

The trend towards worse progression free survival for OC patients harboring the *KRAS*-variant suggests an impact of the *KRAS*-variant on ovarian cancer outcome. Because the *KRAS*-variant is located in the 3'UTR of the *KRAS* oncogene, available tumor samples were tested for *KRAS* codon mutations (*n* = 6 *KRAS*-variant harboring patients, *n* = 10 *KRAS*-variant non-harboring patients). Not surprisingly, as non-mucinous OC rarely has activated *KRAS,* none of the ovarian tumors tested had the common *KRAS* activating mutations. These findings agree with our prior findings that the *KRAS*-variant is not enriched in tumors with other tumor-acquired *KRAS* mutations.

### Association of the KRAS-variant with HBOC Syndrome

As the KRAS-variant appeared to be associated with OC risk for sporadic OC, to further validate its role as a genetic marker of ovarian cancer, OC patients considered to be at high-risk for having a familial genetic abnormality with a family history consistent with HBOC were examined. These patients had either personal and/or family histories (1 st or 2nd degree relatives) of at least one. additional case of OC and/or breast cancer, and all had undergone *BRCA* mutation analysis. 67 patients fit these parameters: 23 were positive for *BRCA1* mutations; 13 were positive for *BRCA2* mutations; and 31 were uninformative (*BRCA1* and -2 mutation negative). Overall 8/36 (22%) of *BRCA* mutation carriers had the *KRAS*-variant: 7/23 (30%) of *BRCA1* mutant carriers and 1/13 (8%) of *BRCA2* mutant carriers. The differential association of the *KRAS*-variant with *BRCA1* and *BRCA2* may represent a biological modification of *BRCA* penetrance by the *KRAS*-variant, a hypothesis that requires additional study.

Of the 31 uninformative HBOC patients with OC, 19/31, or 61% harbored the *KRAS*-variant, a prevalence significantly higher than documented rates for either the healthy population 14 or other OC patients (*p* <0.0001 compared to control patients). For a *KRAS*-variant harboring uninformative HBOC family member this results in a positive predictive value (PPV) for developing OC of 6.78% (95% CI = 5.78 to 7.76). In contrast, the negative predictive value (NPV) for a negative KRAS-variant test in an uninformative HBOC family member is 99.37% (95% CI = 99.22 to 99.53). *KRAS-variant Harboring Families*

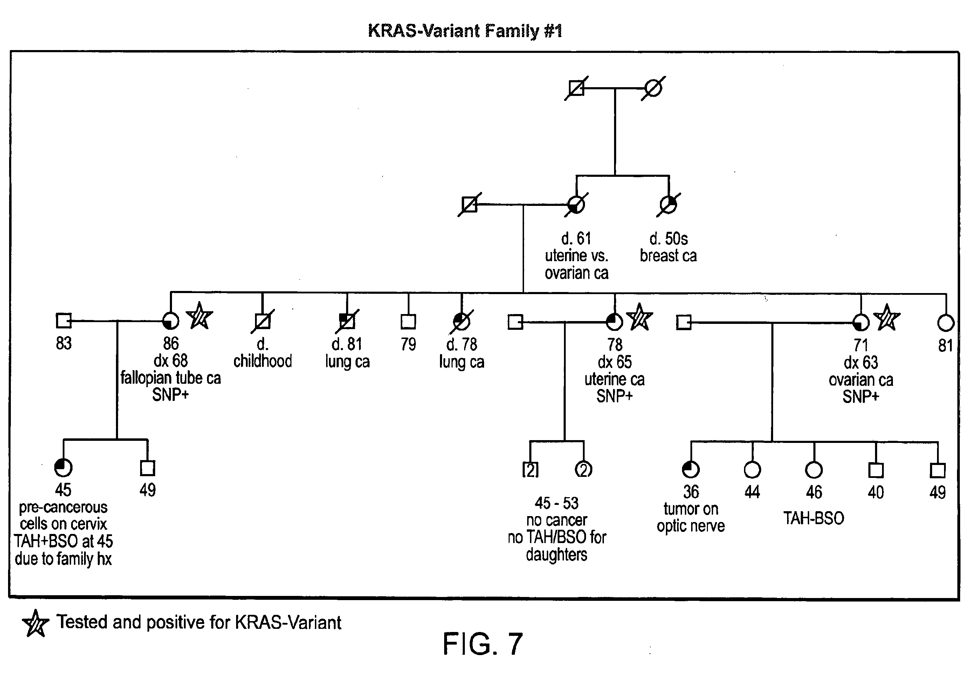

At least two additional family members with known cancer status were tested in four of the uninformative HBOC families whose proband harbored the *KRAS*-variant and was *BRCA* negative. In each of these families, at least two relatives diagnosed with cancer also harbored the *KRAS*-variant (Figure 7 and Table 7). Finally, we compared the pedigrees of HBOC families where we had complete data with a *BRCA1* mutation (*n* = 11), a *BRCA2* mutation (*n* = 8), or the *KRAS*-variant (*n* = 13), and recorded demographics and cancer types in their family members. We found that there are unique familial profiles for each of these groups, which differ by ethnicity, cancer type, and age of cancer onset, with *KRAS*-variant carrying families being significantly more likely to be non-Jewish, have lung cancer in the family, and be older at the time of their OC diagnosis than *BRCA* mutant OC patients (Figure 4B).

### Conclusions

These results reveal that the variant allele at a polymorphism in the *KRAS* 3' UTR, the *KRAS*-variant, is associated with the risk of developing epithelial OC (OR = 2.46), is identified in over 25% of non-selected OC patients and is found in 61% of OC patients from HBOC families previously considered uninformative for gene mutations. These findings support the hypothesis that the *KRAS*-variant is a new genetic marker of an increased risk of developing OC, and, additionally, suggest that this allele of *KRAS* may be a new HBOC locus.

While it may seem surprising that a single nucleotide variant could have such predictive power for disease risk, the *KRAS*-variant represents an entirely different entity than tagging SNPs studied and employed in genome wide association studies. The *KRAS*-variant is not present on Illumina SNP arrays (being recently discovered and failing design), but rather was identified through a candidate-gene search. It is functional and disrupts a *let-7* miRNA binding site that regulates the important human oncogene, *KRAS.* Perhaps most importantly, the *KRAS*-variant is an uncommon allele, being in less than 7% of chromosomes in any ethnic group, and would therefore not be meaningfully detected in GWAS studies through LD with more common alleles.

The OC in *KRAS*-variant carriers has a similar phenotype to the majority of epithelial OC, and occurs primarily in post-menopausal women. This is unlike OC associated with previously identified inherited genetic markers of OC risk, such *as BRCA* mutations, which disrupt DNA repair pathway genes, and are associated with early onset cancer. This suggests that the *KRAS*-variant may not act through altered DNA repair, but perhaps instead creates an environment where alterations that occur normally with aging allow aberrant cell growth and oncogenesis. In support of this hypothesis, we previously reported that the *KRAS*-variant is associated with increased *KRAS* levels in the background of lower *let-7* levels, and others have shown that *let-7* levels decrease with age. While *KRAS* mutations have not been associated with non-mucinous epithelial OC, the *KRAS*-variant may represent a novel form of *KRAS* activation, or lead to disruption of the EGFR-signaling pathway, a pathway frequently misregulated in OC. These hypotheses require further validation in OC tumor tissues, a resource that was not available in these studies.

The frequent association of the *KRAS*-variant with these patients and their family members with cancer suggests that the *KRAS*-variant is a genetic marker of ovarian cancer risk. Identification of new such markers of ovarian cancer risk is critical for these uninformative families, as those who test positive in these families will have a confirmed increased inherited risk, while those who test negative will in fact be at a decreased risk of developing ovarian cancer compared to the general female population, information that will be equally valuable in their management.
Genetic risk factors for cancer have been historically very difficult to identify, and those that are known are found in very few patients and make up a small minority of cancer cases. Because the 3' UTR of a gene is a critical regulatory region likely to be bound by multiple miRNAs, we have proposed that this region is likely to harbor variants, such as the *KRAS*-variant, that may be associated with a large proportion of cancer cases, and be as powerful as gene coding mutations in shaping disease risk.

### Methods

### Samples from New Haven, CT USA

Samples from patients with OC at Yale/New Haven Hospital were recruited and collected from fresh frozen tissue (*n* = 12), paraffin embedded formalin fixed tissue (*n* = 23), blood (*n* = 71) or saliva (*n* = 51) between 2007 and 2009 (total *n* = 157). Since we have previously extensively validated that the *KRAS*-variant is not somatic but germline (identical in patients' normal and tumor tissues), primarily germline DNA was collected in these studies from either blood or saliva. Patient data was collected including age, ethicity and family history of cancer.

OC subtype was established by pathologic classification, with only epithelial OC cases included in this study.

OC patients from HBOC families were recruited through the Yale Cancer Center Department of Genetics, and one individual was included from each family as the index case for statistical analysis.

Controls (all female) were recruited from Yale/New Haven Hospital beginning in 2008 from healthy friends and associates of patients, none were genetically related to the patient. All control DNA samples were derived from saliva. None of the controls had any prior diagnosis of cancer (other than nonmelanoma skin cancer).

Information regarding age, ethnicity and family history was recorded.

### Samples from Turin, Italy

Between October 1991 and February 2000, there were 264 patients who underwent surgery for ovarian tumors at the department of Gynecology, Gynecologic Oncology Unit, at the University of Turin in Italy, and tissue was collected after institutional IRB approval. All patients were Caucasian. Of these patients, 23 were diagnosed with metastatic cancer, 19 with benign tumors, 6 with OC of nonepithelial origin, and 1 with endometriosis. Epithelial ovarian tumors from the remaining 215 patients were included in this study. Additional details on these samples are available. *Samples from Brescia, Italy*

Tumor samples for DNA extraction were collected from 100 patients with epithelial OC at the Division of Gynecologic Oncology at University of Brescia, Italy, between September 2001 and December 2008 after institutional IRB approval. All patients were Caucasian. Clinical data were collected from medical records and were available for all patients. Fifty-nine patients were followed from the date of first surgery until death or May 5, 2009. Patients who received neoadjuvant chemotherapy were excluded from non-static parameters such as debulking, residual disease and PFS.

### Case Control Analysis

The Yale Cases and Controls were selected from those with complete information from Yale/New Haven Hospital (*n* = 100 and 101 respectively). All were women, and were matched for age and ethnicity. For controls who had their ovaries removed for benign reasons, their age at ovarian removal was recorded as their age of testing for this study.

The Connecticut Case-Control study was approved by the Connecticut Department of Public Health and all 32 hospitals that participated. Potential cases were English-speaking women from Connecticut, diagnosed at 35-79 years of age with OC between September 1, 1998 and February 28, 2003, with new primary invasive epithelial ovarian tumors. Controls were a representative sample of the general population of the study area and identified by list-based random digit dialing methods. Cases and controls were matched for age and ethnicity. Cases and controls with prior cancer were excluded from the analysis. Further details are available. Samples used in this study included 320 cases and 328 controls.

### Statistical Methods

For numerical variables (such as age), linear models were used to compare the differences between case and control groups. Chi-square and exact methods were performed to determine the distribution of ethnicity in cases and controls. Hardy-Weinberg testing was analyzed using the ALLELE procedure. Survival analyses were performed using Cox proportional hazards regression model. The association of the *KRAS*-variant with OC was determined using logistic regression modeling. All statistical analyses were performed using SAS version 9.1.2 (SAS Institute, Cary, NC).

### Delecting the Presence of the KRAS-variant

DNA was collected using standard isolation methods from tissue, blood, buccal cell samples or saliva. Only the Connecticut Case Control underwent DNA amplification prior to testing. The *KRAS*-variant was assayed using a allele specific primer and a PCR based Taqman assay using standard techniques. Validation of this assay through duplicate testing and sequencing was previously performed and reported. The *KRAS*-variant is almost always in the heterozygous state in its carriers, with less then 3-5% of any population containing the variant in the homozygous form. The two genotypes that were combined in this work together as positive for the *KRAS*-variant.

### Culculating Positive Predictive Value (PPV)

The PPV is calculated by comparing the percent of *KRAS*-variant positive and negative patients with ovarian cancer and without and multiplying by a lifetime risk of 1.4% of developing ovarian cancer, to determine the difference in lifetime cancer risk. Control prevalence is based on the Yale controls. PPV is then the lifetime cancer risk of *KRAS*-variant positive patients with ovarian cancer over the total *KRAS*-variant people.

**Table 7: Prevalence of the KRAS-Variant in Patients, Ovarian Cancer (OC) presentation, Treatment Response, and Progression Free Survival.**

| **A** | | | |
|---|---|---|---|
| ***KRAS* Genotype** | ***n*** | **Age** | |
| | | **Median** | **Range (Min-Max)** |
| **T/T** | 200 | 58.5 | 24.0-82.1 |
| **G/T and G/G** | 68 | 56.4 | 37.4-80.2 |
| **p value** | | 0.478 | |

| **B** | | | |
|---|---|---|---|
| **Variables** | ***n*** | ***KRAS* Genotype (%)** | |
| | | **T/T** | **G/T and G/G** |
| **Tumor grade** | | | |
| 1-2 | 84 | 64 (76.2) | 20 (23.8) |
| 3 | 186 | 138 (74.2) | 48 (25.8) |
| p value | | 0.726 | |

| **Residual tumor size** | | | |
|---|---|---|---|
| 0 | 91 | 68 (74.7) | 23 (25.3) |
| >0 | 116 | 86 (74.1) | 30 (25.9) |
| p value | | 0.923 | |

| **Debulking results** | | | |
|---|---|---|---|
| Optimal | 108 | 81 (75.0) | 27 (25.0) |
| Suboptimal | 100 | 73 (73.0) | 27 (27.0) |
| p value | | 0.742 | |

| **C** | | | |
|---|---|---|---|
| **Variables** | ***n*** | ***KRAS* Genotype (%)** | |
| | | **T/T** | **G/T and G/G** |
| **Disease stage** | | | |
| I-II | 64 | 51 (79.7) | 13 (20.3) |
| III-IV | 147 | 106(72.1) | 41 (27.9) |
| p value | | 0.246 | |

| **D** | | | |
|---|---|---|---|
| **Variables** | ***n*** | **KRAS Genotype (%)** | |
| | | **T/T** | **G/T and G/G** |
| **Treatment response** | | | |
| No | 74 | 52 (70.3) | 22 (29 7) |
| Yes | 183 | 139 (76.0) | 44 (24.0) |
| p value | | 0.345 | |

| **E** | | | |
|---|---|---|---|
| ***KRAS* Genotype** | **Progression** | | |
| | HR | 95% CI | |
| **Unvariate analysis** | | | |
| **T/T** | 1.00 | | |
| **G/T and G/G** | 1.12 | 0.71-1.76 | |

| | | | |
|---|---|---|---|
| G/T represents heterozygous and G/G homozygous for the KRAS-Variant allele. n is number of patient samples per category. A. KRAS-Variant harboring patients are a similar age to non-KRAS-variant patients. B. Pathologic variables including grade, size, and surgical debulking are not significantly different between KRAS-Variant non-harboring and harboring patients. C. KRAS-variant harboring patients are slightly more likely to present with advanced disease. D. There is a non-significant trend for KRAS-variant harboring patients to not respond to therapy. E. There is a trend for KRAS-variant harboring patients to have worse progression free survival. | | | |

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method of predicting an increased risk of hereditary breast/ovarian cancer syndrome (HBOC syndrome) in a subject, comprising detecting a single nucleotide polymorphism (SNP) at position 4 of the let-7 complementary site 6 of KRAS, corresponding to position 4 of SEQ ID NO:6, in a patient sample, wherein said subject is BRCA1 or BRCA2 mutation positive, and wherein the presence of said SNP indicates an increased risk of HBOC syndrome in said subject.

2. The method of claim 1, further comprising the step of detecting a BRCA1 mutation in said subject, wherein the presence of said BRCA1 mutation and said SNP indicates an increased risk of developing HBOC syndrome.

3. The method of claim 1 or claim 2, wherein the presence of said BRCA1 mutation and said SNP indicates an increased risk of developing breast cancer or ovarian cancer.

4. The method of claim 1, 2 or 3, wherein said BRCA1 mutation is a non-founder mutation.

5. The method of claim 1 or 2, wherein the presence of said BRCA1 mutation and said SNP indicates an increased risk of developing both breast and ovarian cancer.

6. The method of claim 5, wherein said BRCA1 mutation is a non-founder mutation.

## Patentansprüche

1. Verfahren zur Vorhersage eines erhöhten Risikos für erbliches Brust-/Ovarialkrebs-Syndrom (HBOC-Syndrom) bei einem Patienten, umfassend den Nachweis eines Einzelnukleotidpolymorphismus (SNP) an Position 4 der zu let-7 komplementären Stelle 6 von KRAS, entsprechend Position 4 von SEQ ID NO:6, in einer Patientenprobe, wobei der Patient für die BRCA1- oder BRCA2-Mutation positiv ist, und wobei das Vorliegen des SNP ein erhöhtes Risiko für HBOC-Syndrom bei dem Patienten anzeigt.

2. Verfahren nach Anspruch 1, weiterhin umfassend den Schritt des Nachweises einer BRCA1-Mutation bei dem Patienten, wobei das Vorliegen der BRCA1-Mutation und des SNP ein erhöhtes Risiko für die Entwicklung von HBOC-Syndrom anzeigt.

3. Verfahren nach Anspruch 1 oder Anspruch 2, wobei das Vorliegen der BRCA1-Mutation und des SNP ein erhöhtes Risiko für die Entwicklung von Brustkrebs oder Ovarialkrebs anzeigt.

4. Verfahren nach Anspruch 1, 2 oder 3, wobei die BRCA1-Mutation eine Nicht-Gründermutation ist.

5. Verfahren nach Anspruch 1 oder Anspruch 2, wobei das Vorliegen der BRCA1-Mutation und des SNP ein erhöhtes Risiko für die Entwicklung von sowohl Brust-als auch Ovarialkrebs anzeigt.

6. Verfahren nach Anspruch 5, wobei die BRCA1-Mutation eine Nicht-Gründermutation ist.

## Revendications

1. Procédé pour prédire un risque accru de syndrome héréditaire du cancer du sein et de l'ovaire (syndrome HBOC) chez un sujet, comprenant la détection d'un polymorphisme d'un seul nucléotide (SNP) en position 4 du site 6 complémentaire de let-7 de KRAS, correspondant à la position 4 de la SEQ ID NO : 6, dans un échantillon provenant d'une patiente, dans lequel ledit sujet est positif à une mutation BRCA1 ou BRCA2, et dans lequel la présence dudit SNP indique un risque accru de syndrome HBOC chez ledit sujet.

2. Procédé selon la revendication 1, comprenant en outre l'étape de détection d'une mutation BRCA1 chez ledit sujet, dans lequel la présence de ladite mutation BRCA1 et dudit SNP indique un risque accru de développer un syndrome HBOC.

3. Procédé selon la revendication 1 ou la revendication 2, dans lequel la présence de ladite mutation BRCA1 et dudit SNP indique un risque accru de développer un cancer du sein ou un cancer de l'ovaire.

4. Procédé selon la revendication 1, 2 ou 3, dans lequel ladite mutation BRCA1 est une mutation non fondatrice.

5. Procédé selon la revendication 1 ou 2, dans lequel la présence de ladite mutation BRCA1 et dudit SNP indique un risque accru de développer à la fois un cancer du sein et un cancer de l'ovaire.

6. Procédé selon la revendication 5, dans lequel ladite mutation BRCA1 est une mutation non fondatrice.
